# EUROPEAN PATENT APPLICATION

(11) **EP 1 493 808 A1**
(43) Date of publication of application: **05.01.2005**
(21) Application number: 03077103.4
(22) Date of filing: 03.07.2003
(51) Int. Cl.: C12N 15/00

(54) **Vectors based on novel plasmids derived from Lactobacillus plantarum**

(71) Applicant: Wageningen Centre for Food Sciences, 6700 AN Wageningen (NL)
(72) Inventor: Van Kranenburg, Richard, 6708 DE Wageningen (NL); Bongers, Roger Samuel, 6714 DN Ede (NL); Siezen, Roelant Jan, 6711 TE Ede (NL); De Vos, Willem Meindert, 6721 SJ Bennekom (NL); Kleerebezem, Michiel, 6716 GG Ede (NL)
(74) Representative: Jorritsma, Ruurd

(57) **Abstract**

The present invention relates to replicons from plasmids found in *Lactobacillus plantarum* strain WCFS 1. The replicons are used for the construction of cloning vectors for use in *Lactobacillus plantarum* and related species. The vectors may be used to introduce and express DNA fragments of interest in lactic acid bacterial host cells. The transformed host cells are useful in various fermentation processes, including processes for the production of food products. The vectors are particularly useful for self-cloning in *Lactobacillus plantarum*.

## Description

### Field of the invention

The present invention relates to novel cloning vectors for *Lactobacillus plantarum* and related species. The vectors are based on replicons derived from plasmids that occur naturally in *L. plantarum.* The vectors may be used to introduce and express DNA fragments of interest in lactic acid bacterial host cells. The transformed host cells are useful in various fermentation processes, including processes for the production of food products.

### Background of the invention

Lactic acid bacteria are used for the preservation of food and feed raw materials like milk, meat, and vegetables or other plant materials. In addition, certain strains of lactic acid bacteria, in particular strains from the genera *Lactobacillus* have been attributed probiotic activities in man and animals (Kalliomaki et al., 2001). Several lactic acid bacteria, including lactococci, lactobacilli, and pediococci, are known to harbour plasmids. These plasmids may encode important traits like resistance to phages or antibiotics, lactose catabolism, and production of proteolytic enzymes or bacteriocins.

*Lactobacillus plantarum* species often harbour several plasmids (Ruiz-Barba et al., 1991). Only a few of the *L. plantarum* plasmids have been characterised in some more detail and sequenced (Bates and Gilbert, 1989, Danielsen, 2002, Eguchi et al., 2000, Kanakeo et al., 2000, Leer et al., 1992, Skaugen, 1989, Vujcic and Topisirovic, 1993). For most of the *L. plantarum* plasmids it is not known which functions they encode. However, two *L. plantarum* plasmids encoding phage resistance and tetracycline resistance have been described (Danielsen, 2002, Eguchi et al., 2000). All plasmids found in *L. plantarum* thus far are smaller than 11 kb and are predicted to replicate via the rolling-circle replication (RCR) mechanism, except for plasmid pMD5057 that is predicted to replicate via the theta mechanism (Danielsen, 2002).

The capacity for conjugal transfer is an important characteristic for plasmids. Self-transmissible conjugative plasmids have the ability to form effective cell-to-cell contact, while mobilisable plasmids are only able to prepare their DNA for transfer (Lanka and Wilkins, 1995). Mobilisation involves the action of a specific DNA-protein structure called the relaxosome to produce single-stranded cleavage at the nicking site (*nic*) within the origin of transfer (*oriT*) of the plasmid (Lanka and Wilkins, 1995). To date there is very little information on conjugation in Lactobacilli. Ahn et al. describe an 8.5-kb chloramphenicol resistance plasmid, which has been comobilised with the broad host-range conjugative plasmid pAMβ1 from *L. plantarum* to *Carnobacterium piscicola* (Ahn et al., 1992). To our knowledge no conjugative *L. plantarum* plasmids have been reported.

It is an object of the present invention to provide novel cloning vectors with improved properties. The novel cloning vectors are based on replicons that are derived from three novel natural *L. plantarum* plasmids.

### Description of the invention

### Definitions

The term "plasmid" as used herein refers to an extrachromosomal genetic element. Plasmids are DNA molecules that can vary in size from 1 kb to more than 200 kb. Most plasmids consist as double-stranded, covalently closed, circular (CCC) DNA molecules that can be isolated from bacterial cells in superhelical form. Plasmids are found to naturally occur in a wide variety of bacterial species. Most plasmids have a narrow host range and can be maintained only in a limited set of closely related species. Other plasmids have a broader host range and are stably maintained in a wider variety of bacterial genera. Plasmids are extrachromosomal elements that behave as accessory genetic units that autonomously replicate and are inherited independently from the bacterial chromosome. Plasmids have evolved a variety of mechanisms to maintain a stable copy number of the plasmid in the bacterial host cell and to partition plasmid molecules accurately to daughter cells. Plasmids usually depend, to a greater or lesser extent, on chromosomally encoded enzymes and proteins for their replication, transcription and translation. Frequently, plasmids contain genes that are advantageous to the bacterial host cell. Phenotypes that are conferred by naturally plasmids include resistance to and production of antibiotics, resistance to heavy metals, virulence factors, the ability to metabolise specific carbohydrates, and restriction and modification enzymes. Although plasmids are mostly extrachromosomal, the term plasmid does not exclude molecules that integrate into the bacterial chromosome, at varying frequency.

The term "replicon" is used herein to define the smallest fragment of DNA that is able to replicate autonomously and, preferably, maintain normal copy number. A replicon is thus a genetic unit consisting of an origin of DNA replication and its associated control elements. In plasmids, the origin of replication is usually a defined segment of DNA, several hundreds base pairs in length. Its set of associated cis-acting controlling elements contain sites for diffusible plasmid- and host-encoded factors involved in initiation of DNA synthesis.

The term "vector" as used herein refers to a nucleic acid compound used for introducing exogenous DNA into host cells. A plasmid vector thus at least comprises a replicon and a DNA segment that may be used for insertion of the exogenous DNA into the vector by recombinant techniques, preferably without interfering with the plasmids capability to replicate. Vectors usually comprise further genetic elements to facilitate their use in molecular cloning, such as e.g. selectable markers, multiple cloning sites and the like (see below). An "expression vector" as used herein refers to any plasmid-based cloning vector, in which a promoter and other regulatory elements are present to enable transcription of inserted exogenous DNA when the expression vector is present in a suitable host cell. A "shuttle vector" refers to a plasmid vector that is capable of replication and stable maintenance in at least two different host organisms, e.g. two organisms of different species or different genera. For this capability the shuttle vector may rely on a single broad host-range replicon but usually a shuttle vector will comprise different replicons for different host-organisms or different groups of host-organisms.

The term "selectable marker" is a term familiar to one of ordinary skill in the art and is used herein to describe any genetic entity which, when expressed, can be used to select for a cell or cells containing the selectable marker. For selection of the cells containing the selectable marker growth conditions are applied that allow growth of cells containing the selectable marker and that inhibit growth of cells lacking the selectable marker gene. The selective pressure exerted by the combination of growth conditions and selective marker, thus allows to select for host cells that have taken up the transforming DNA from untransformed cells, or, once tranformed cells have been obtained, the selective pressure may be used to provide a growth advantage to cells retaining the marker over cells that have lost the transforming DNA. The selectable marker can be dominant with respect to the host cells, which means that the marker can be used in wild-type host cells, i.e. without prior genetic modification of the host cells. Examples of dominant markers are e.g. genes providing resistance to antibiotic or heavy metals. The selective marker may also be recessive with respect to the host cells, which means that endogenous genes having the same activity as the marker need to be inactivated before the marker can be used in the host cells. Examples of such genes include genes which confers prototrophy to an auxotrophic strain, i.e. auxotrophic markers, which usually encode an enzyme which is at least partially responsible for the synthesis of a metabolite which is necessary for the organism containing the selectable marker to grow and survive in a medium that does not contain the particular metabolite. Yet another type of marker is the bidirectional selection marker, for which both a positive and a negative genetic selection is possible. The postive selection applies conditions that select for cells containing the bidirectional marker whereas the negative selection applies different conditions that select for cells lacking or having lost the bidirectional marker.

The term "homologous" when used to indicate the relation between a given (recombinant) nucleic acid or polypeptide molecule and a given host organism or host cell, is understood to mean that in nature the nucleic acid or polypeptide molecule is produced by a host cell or organisms of the same species, preferably of the same variety or strain. If homologous to a host cell, a nucleic acid sequence encoding a polypeptide will typically be operably linked to another promoter sequence or, if applicable, another secretory signal sequence and/or terminator sequence than in its natural environment. Such regularory sequences may also be homologous to the host cell. In this context, the use of only "homologous" sequence elements allows the contruction of "self-cloned" genetically modidified organisms (GMO's).

When used to indicate the relatedness of to nucleic acid sequences the term "homologous" means that one single-stranded nucleic acid sequence may hybridize to a complementary single-stranded nucleic acid sequence. The degree of hybridization may depend on a number of factors including the amount of identity between the sequences and the hybridization conditions such as temperature and salt concentration as discussed later. Preferably the region of identity is greater than about 5 bp, more preferably the region of identity is greater than 10 bp.

### Detailed description of the invention

In a first aspect the present invention relates to an (isolated) nucleic acid molecule comprising a replicon derived from pWCFS101, pWCFS102 or pWCFS103. The replicon preferably comprises all the sequence elements that are necessary in cis for replication of a plasmid containing the replicon as sole replicon, in a Lactobacillus host cell, preferably a *L. plantarum* host cell. In as far as necessary for replication, the various cis-acting sequence elements of the replicons are operably linked.

All the cis-acting sequence elements that are necessary for replication of a plasmid are present in the *Xba*I fragment of pWCFS101, which is depiceted in SEQ ID NO: 1, and is e.g. present in pR706. Methods for determining whether this fragment contains additional sequences that are not necessary for replcation are know per se to the skilled person. Replicons from which such non-essential sequences have removed are part of the present invention. The replicon derived from pWCFS101 preferably comprises a sequence coding for the 37 kDa RepA replication protein of 319 amino acids, whereby the coding sequence preferably is operably linked to appropriate regulatory sequences for its expression. However, the 37 kDa RepA replication protein may be provided in trans and therefore its coding sequence is not necessarily part of the replicon derived from pWCFS101. In such instances the 37 kDa RepA coding sequence may be present in another postion in the plasmid, on a second plasmid and/or integrated in the genome of the host cell.

All the cis-acting sequence elements that are necessary for replication of a plasmid are present in the *Xba*I fragment of pWCFS102, which is depiceted in SEQ ID NO: 2, and is e.g. present in pR707. Methods for determining whether this fragment contains additional sequences that are not necessary for replcation are know per se to the skilled person. Replicons from which such non-essential sequences have removed are part of the present invention. The replicon derived from pWCFS102 preferably comprises sequences coding for the 6 kDa RepA replication protein of 52 amino acids and the 25 kDa RepB replication protein of 266 amino acids, whereby the coding sequences preferably are operably linked to appropriate regulatory sequences for their expression. However, the 25 kDa RepB replication protein may be provided in trans and therefore its coding sequence is not necessarily part of the replicon derived from pWCFS101. In such instances the 25 kDa RepB coding sequence may be present in another postion in the plasmid, on a second plasmid and/or integrated in the genome of the host cell.

All the cis-acting sequence elements that are necessary for replication of a plasmid are present in the *Xho*I*-Sal*I fragment that may be amplified with primers as depicted in SEQ ID NO's: 7 and 8 using pWCFS103 as template, and as e.g. present in pR701. The sequence of this fragment is the sequence from postion 260 to position 3410 in SEQ ID NO: 3. Methods for determining whether this fragment contains additional sequences that are not necessary for replication are know per se to the skilled person. Replicons from which such non-essential sequences have removed are part of the present invention. The replicon derived from pWCFS103 preferably comprises sequences coding for the 12 kDa RepA replication protein of 102 amino acids and the 30 kDa RepB replication protein of 266 amino acids, whereby the coding sequences preferably are operably linked to appropriate regulatory sequences for their expression. The replicon derived from pWCFS103, further preferably at least comprises a sequence coding for the replication protein of 116 amino acids encoded by *orf4,* whereby the coding sequence preferably is operably linked to appropriate regulatory sequences for its expression, or whereby the coding sequence is operably linked to the coding sequence of RepB. However, the 12 kDa RepA, 25 kDa RepB and *orf4* replication proteins may be provided in trans and therefore their coding sequences are not necessarily part of the replicon derived from pWCFS103. In such instances the 12 kDa RepA, 25 kDa RepB and *orf4* coding sequences may be present in another postion in the plasmid, on a second plasmid and/or integrated in the genome of the host cell.

The above described replicons derived from pWCFS101, pWCFS102, and pWCFS103 may be obtained as nucleic acid fragments comprising or consisting of the nucleotide sequences depicted in, respectively SEQ ID NO's: 1, 2, or 3, respectively.

In a preferred embodiment, the nucleic acid molecule comprising a replicon as defined above, is a plasmid.

In a further aspect the invention relates to a vector comprising a plasmid as defined in above. Preferably the vector comprises a coding sequence that, upon expression, confers to a host cell containing the vector a phenotype that allows to discriminate a host cell containing the vector from host cells that are genetically identical except that they do not contain the vector. The coding sequence preferably is operably linked to regulatory sequences that are capable of driving expression of the coding sequence when the plasmid is contained in a suitable host cell. The coding sequence providing the distinguishable phenotype may thus be a gene that allows screening for host cells containing the vector using a suitable assay, such as e.g. colour or fluorescence. Suitable genes for this purpose are e.g. genes encoding a β-galactosidase (e.g. the *E. coli lacZ* gene), a β-glucuronidase, a Green Fluorescent Protein (GFP) or a variant therof and the firelfly luciferase. Preferably, however, the gene for discrimination of host cells containing the vector encodes a selectable marker.

The gene encoding a selectable marker may encode a dominant, a recessive or a bidirectional selectable marker as described above. Preferred selectable marker genes are homologous to the host cell. Suitable dominant selectable markers are genes providing antibiotic resistance, heavy metal resistance or the capability to utilise certain sugars. Examples thereof include e.g. genes conferring resistance to antibiotics such as kanamycins, rifampicin, erythromycin, actinomycin, chloramphenicol, tetracyclines, nisin and lactacin F or genes conferring resistance to a heavy metal such as arsenite, arsenate, antimony, cadmium (e.g. Cd^{r} of *L. lactis,* Liu et al., 1996, Curr. Microbiol. 33: 35-39) or organo-mercurial compounds. Such genes are generally known to the skilled person. Preferred dominant markers are food-grade markers such as the sugar utilisation genes *xylRAB* from *Lb. pentosus* (Posno et al., 1991, Appl. Environ. Microbiol. 57: 2764-2766), *levA* from *Lb. plantarum* (Wanker et al., 1994, Appl. Environ. Microbiol. 60: 1401-1413) or *Lb. casei* (Wanker et al., 1995, Appl. Microbiol. Biotechnol. 43: 297-303) and *scrA*/*scrB* from *P. pentosaceus* (Leenhouts et al., 1998, Appl. Microbiol. Biotechnol. 49: 417-423).

Recessive selectable markers for use in the present invention include e.g. genes which confers prototrophy to an auxotrophic strain, so-called auxotrophic markers. Examples of suitable food grade auxotrophic markers include e.g. tRNA (gln) of *L*. *lactis* (Dickely et al., 1995, Mol. Microbiol. 15: 839-847) and *alr* of *L. lactis* or *Lb plantarum* (Bron et al., 2002, Appl. Environ. Microbiol. 68: 5663-5670).

An example of a suitable bidirectional marker for use in the present invention is a gene encoding a orotidine-5'-decaboxylases which is to be used in host lacking endogenous orotidine-5'-decaboxylase. Host cells containing a vector encoded orotidine-5'-decarboxylase can then be selected in a (mineral) medium lacking uracil, whereas such host cells can be cured from vectors encoding a orotidine-5'-decarboxylase by counter-selection on a medium containing 5-fluoro-orothic acid. Similarly, a gene encoding an acetamidase can be used as bidirectional marker by selection on a medium containing acetamide as sole carbon and or nitrogen source and by counter-selection on a medium containing fluoro-acetamide (see EP 0 635 574). An additional advantage of acetamidase markers is that with respect to most bacterial host cells they are dominant selectable markers, which do not require a mutant (auxotrophic) strain, but which can be used directly in wild type strains.

The vectors and plasmids of the invention may further comprise sequences for promoting conjugation or other means of transfer of genetic material. Preferably such sequences are homologous to the host cell. Examples of such sequences include e.g. the mobilisation gene cluster of the *Lactococcus lactis* plasmid pMRC01. This *L. lactis* Tra region comprises an origin of transfer (*oriT*) sequence flanked by a gene cluster of 18 genes (*traA, orf6, orf7, traB, traC, traD, traE, traF, orf13, orf14, orf15, traJ, traK, orf18, traL, traI, orf21, ltrC*), and a gene cluster of two genes in the opposite orientation (*orf 4* and *orf3*) (Dougherty *et al.,* 1998). A preferred mobilisation gene cluster is actually found in pWCFS103 of the present invention. The Tra region of pWCFS103 shares homology with the *L. lactis* Tra and runs from *orf37* (*orf3* in pMRC01) to *orf17* (*ltrC* in pMRC01) and is flanked by two transposase genes (Table 2). The pWCFS103 Tra region has an additional ORF, *orf20,* of unknown function in between *traL* and *traI.* The pWCFS103 *oriT* region contains the CGAAG sequence, which is conserved among several conjugative plasmids (Dougherty et al., 1998, Wang and Macrina, 1995) and which is preceded by a TTAAG sequence. Thus in a preferred embodiment, the vectors and plasmids of the invention comprise a mobilisation gene cluster from pWCFS103 that comprises at least the DNA sequence from 13100 to 30584.

The vectors and plasmids of the invention may further comprise a second replicon different from the replicons of the invention as described above. The purpose of the second replicon is to expand the host-range for the vectors and plasmids of the invention. Such a vector or plasmid containing two different replicons may thus be shuttled between two or more different types of host cells. A particularly preferred second replicon is a replicon that is capable of replication in *Escherichia coli,* so as to facilitate multiplication and manipulation in molecular cloning. Suitable replicons for *E. coli* are well known to the skilled molecular biologist and include e.g. pMB1 or a modified form thereof, pKN402, p15A, pSC101, and colE1 (see Sambrook and Russell (2001) "Molecular Cloning: A Laboratory Manual (3^{rd} edition), Cold Spring Harbor Laboratory, Cold Spring Harbor Laboratory Press, New York).

Vectors of the invention further preferably comprise one or more restriction sites that are suitable for insertion of desired DNA fragments. Restriction sites that are suitable for this purpose preferably are unique in the vector, allowing to linearise the vector into a single DNA fragment corresponding to the complete vector. Such restriction sites further preferably are not present in DNA sequences that are required for replication, maintenance or selection. The vectors may further comprise a multiple cloning site, as is known in the art. Such multiple cloning site contains several different unique restriction sites that may conveniently be used for insertion of fragments into the vectors.

In a further embodiment, the vector comprises a promoter that, when the vector is present in a suitable host cell, is capable of driving transcription of a nucleotide sequence of interest operably linked to the promoter. In principle, any suitable constitutive or inducible promoter that is active in the host cell may be used for this purpose. Preferred promoters are homologous to the host cell. Examples of suitable constitutive promoters are the *usp45* promoter (van Asseldonk et al., 1990, Gene, 95: 155-160), the *nisR* promoter (de Ruyter et al., 1997, J. Bacteriol., 178: 3434-3439), the *pepN* promoter (Tan et al., 1992, FEBS Lett., 306: 9-16) and the promoters mentioned in de Vos and Simons in "Genetics and Biotechnology of Lactic Acid Bacteria", Gasson and de Vos, eds., pp. 52-106, Chapman and Hall, 1994. Examples of suitable regulated promoters are the bacteriophage *31* middle promoter and *ori*-based expression system (O'Sullivan et al., 1996, Biotechnology, 14: 82-87), the *xylA* promoter (Lokman et al., 1994, Mol. Gen. Genet., 245: 117-125), the repressor/operator system of bacteriophage *rlt* (Nauta et al., 1996, Mol. Microbiol., 19: 1331-1341), the acid-inducible or acid-responsive F₁ F₀-ATPase promoter of *L. acidophilus* (U.S. Patent No. 6,242,194), the promoters of the lactose operon of *S. thermophilus* and *lac ABCDFEGX* operon of *L. lactis* (see, e.g., Simons et al., 1993, J. Bact. 175:5186-5175; Mollet et al., 1993, J. Bact. 175:4315-4324), and the salt-inducible promoters of *L. lactis* that are disclosed in U.S. Patent No. 6,140,078.

A highly suitable class of promoters are the auto-inducible promoters as described in e.g. Kuipers et al. (1997, Tibtech, 15: 135-140). Non-limiting examples are the promoters from the bacteriocin gene cluster of *Carnobacterium piscicola,* the Sakaricin gene cluster from *L. sake,* as well as (presumably) the Subtilin gene cluster from *Bacillus subtilis.* According to the invention these also include promoters for similar, non-bactericidal signal proteins involved in the quorum sensing process in microorganisms, such as the promoter for Plantaricin A from the bacteriocin gene cluster of *L. plantarum.* Reference is made to Kleerebezem et al. (1997, Mol. Microbiol. 24: 895-904) and the references mentioned therein. The preferred promoters used are the auto-inducible promoters from the nisin gene cluster of *L. lactis,* especially the *nisA* and *nisF* promoters, as described in the EP 0 712 935 (*nis A*) and also in De Ruyter et al. (1996, Appl. Environ. Microbiol. 62: 3662-3667; and Kuipers et al., *supra* and the references given therein). These promoters have already been used for the controlled (over-) expression of homologous and heterologous polypeptides (such as the *gusA* reporter gene from *E. coli, pepN,* and the lytic genes *lytH* and *lytA)* in lactic acid bacteria and for the nisin-induced expression in heterologous hosts such as *L. helveticus* and *Leuconostoc lactis.* Preferably, in the vectors of the invention a unique restriction site or a multiple cloning site is located immediately downstream of the promoter in the direction of transcription from the promoter. Further suitable promoters for use in the nucleic acid molecules of the invention are reviewed by de Vos (1999, Int. Dairy J. 9: 3-10).

Vectors of the invention that comprise a promoter as described above for the expression of a coding sequence of interest preferably comprise additional sequence elements to regulate the expression of the coding sequence, i.e. sequences for initiation of translation, secretion, termination of transcription and the like. Such additional sequence elements for regulation of expression are preferably homologous to the host cell. In the vectors, the promoter may be operably linked to more than one coding sequence, i.e. several coding sequences may be present in a single multicistronic transcription unit regulated by the promoter. The one or more coding sequences operably linked to the promoter are preferably each provided with the appropriate signals for initiation of translation such as e.g. a Shine-Dalgamo sequence and a translation initiation codon. Optionally, a coding sequence in the vector comprises a signal sequence encoding a signal peptide causing translocation of the polypeptide encoded by the coding sequence across the cell membrane, and preferably secretion of the polypeptide from the host cell. Signal sequences are well known to the skilled person and a variety of suitable signal sequences is available in the art. Usually the skilled person will select a signal sequence known to effect secretion of polypeptides from a given host cell of choice. Preferably, the signal sequence to be used in the invention will be homologous to the host organism or will originate from an organism closely related to the host organism. Signal sequences that are preferably used in the context of the present invention include signal sequences from lactic acid bacteria of which preferably *Lactobacillus.* Examples of such signal sequences include e.g. signal sequences from α-amylase, *slpA,* and *prtP.* Further suitable signal sequences may be identified using dedicated genetic screening methods as described by Poquet et al. (1998) and Hols et al. (1992). In the vectors of the invention, the transcription unit comprising the one or more coding sequences linked to the promoter is preferably terminated by a suitable transcription terminator, such e.g. *tldH* or *tslP.* Suitable bacterial transcription terminator sequences may be identified as described by Ermolaeva et al (2000).

In a further embodiment, the vectors of the invention comprise one or more nucleotide sequences of interest. Preferably the nucleotide sequence of interest is a coding sequence coding for a polypeptide of interest. Preferably the nucleotide sequence of interest is homologous to the host cell. The polypeptide of interest may have industrial or medical (pharmaceutical) applications. Examples of proteins or polypeptides with industrial applications include enzymes such as e.g. lipases (e.g. used in the detergent industry), proteases (used inter alia in the detergent industry, in brewing and the like), cell wall degrading enzymes (such as cellulases, pectinases, β-1,3/4- and β-1,6-glucanases, rhamnogalacturonases, mannanases, xylanases, pullulanases, galactanases, esterases and the like, used in fruit processing wine making and the like or in feed), phytases, phospholipases, glycosidases (such as amylases, β-glucosidases, arabinofuranosidases, rhamnosidases, apiosidases and the like), dairy enzymes (e.g. chymosin), mammalian, and preferably human, proteins or polypeptides and/or enzymes with therapeutic, cosmetic or diagnostic applications including e.g. insulin, serum albumin (HSA), lactoferrin, hemoglobin α and β, tissue plasminogen activator (tPA), erythropoietin (EPO), tumor necrosis factors (TNF), BMP (Bone Morphogenic Protein), growth factors (G-CSF, GM-CSF, M-CSF, PDGF, EGF, and the like), peptide hormones (e.g. calcitonin, somatomedin, somatotropin, growth hormones, follicle stimulating hormone (FSH) interleukins (IL-x), interferons (IFN-y). Also included are bacterial and viral antigens, e.g. for use as vaccines, including e.g. cholera (heat-labile) toxin B-subunit, Hepatitis B virus envelope surface protein, Norwalk virus capsid protein, Human cytomegalovirus glycoprotein B, glycoprotein S, interferon, and transmissible gastroenteritis corona virusreceptors and the like. Further preferred coding sequences include sequences coding for mutants or analogues of the polypeptides mentioned above.

In another aspect the invention relates to a bacterial host cell comprising a nucleic acid molecule or a vector as defined above. The bacterial host cell preferably is a Gram-positive bacterium, more preferably a Gram-positive bacterium that belongs to a genus selected from the group consisting of *Lactobacillus, Lactococcus, Leuconostoc, Carnobacterium, Bifidobacterium, Bacillus, Streptococcus.* Most preferably the bacterial host cell is a bacterium that belongs to a species selected from the group consisting of *L. acidophilus, L. amylovorus, L. bavaricus, L. brevis, L. caseii, L. crispatus, L. curvatus, L. delbrueckii, L. delbrueckii* subsp. *bulgaricus, L. fermentum, L. gallinarum, L. gasseri, L. helveticus, L. jensenii, L. johnsonii, L. minutis, , L. murinus L. paracasei, L. plantarum, L. pontis, L. reuteri, L. sacei, L. salivarius, L. sanfrancisco, Lactobacillus ssp., C. piscicola, B.subtilis, Leuconostoc mesenteroides, Leuconoctoc lactis, Leuconostoc ssp, B. bifidum, B. longum, B. infantis, B. breve, B. adolescente, B. animalis, B. gallinarum, B. magnum,* and *B. thermophilum.*

In a preferred embodiment, however, the bacterial host cell is of the same species as the species from which the replicons are derived, i.e. *L. plantarum.* Preferably all further sequences that may be present in the nucleic acid molecule or vector as defined above are also derived from the species *L. plantarum,* i.e. are homologous to *L. plantarum.* Such further sequences may thus include selection markers, promoters and genes of interest as described above and as reviewed by de Vos (1999, *supra*). In case a strain is constructed through genetic engineering such that the resulting recombinant strain comprises only sequences derived from the same species as the strain is, albeit in recombined form, the strain is said to be obtained through "self-cloning". Strains obtained through self-cloning have the advantage that there application in food (or pharmaceuticals) is more readily accepted by the public and regulatory authorities as compared strains comprising foreign nucleic acid sequences. The present invention thus allows the construction of self-cloned *L. plantarum* strain for food, pharmaceutical or nutraceutical applications (see also de Vos, 1999 *supra*).

Nucleic acid molecules with the replicons derived from pWCFS101, pWCFS102, and pWCFS103 each have different copy numbers in exponentially growing host cells under non-selective conditions. A nucleic acid molecule with a replicon derived from pWCFS101 has a copy number of about 11.9 (+/-1.2), as exemplified by p706. A nucleic acid molecule with a replicon derived from pWCFS102 has a copy number of about 2.8 (+/- 0.3), as exemplified by p707. A nucleic acid molecule with a replicon derived from pWCFS103 has a copy number of about 4.7 (+/- 0.4), as exemplified by p701. These differences in copy number between the three different replicons may be used to regulate the gene dosage of a gene of interest. Thus, in a preferred embodiment a replicon derived from pWCFS102 is used in the nucleic acid molecules, host cells and methods of the invention to achieve a copynumber of about 3, a replicon derived from pWCFS103 is used to achieve a copynumber of about 5, and a replicon derived from pWCFS101 is used to achieve a copynumber of about 12.

In another aspect, the invention pertains to a method of transforming a bacterial host cell, the method comprising introducing into the host cell a nucleic acid molecule or a vector as defined above. Methods for introducing DNA into lactic acid bacteria are described in e.g. "Genetics and Biotechnology of Lactic Acid Bacteria", Gasson and de Vos, eds. (1993, *supra*).

In yet another aspect, the invention relates to methods for producing a fermentation product, whereby these methods comprise culturing a bacterial host cell as defined above. The fermentation process may be a process for the production of a fermentation product such as ethanol, lactic acid, acetic acid, succinic acid, amino acids, 1,3-propane-diol, ethylene, glycerol, or products known as nutraceuticals, such as e.g. vitamins, antioxidants or poly- or oligo-saccharides. It may also be a process for the production of a fermented food product such as a fermented dairy, vegetable or meat product. The fermentation process may also be a process for producing a polypeptide of interest, wherein the method comprises culturing a bacterial host cell as defined above, under conditions conducive to the expression of the polypeptide. Optionally these methods comprise the step of recovery of the fermentation product or polypeptide of interest.

In yet a further aspect, the invention relates to a method for site-specific delivery of a polypeptide to the gastrointestinal tract of a subject. Preferably the polypeptide is a therapeutic polypeptide. The method comprises administering a bacterial host cell as defined above to the gastrointestinal tract of the subject. The bacterium is preferably orally administered in a composition that is suitable for consumption. A preferred embodiment of such method is a method for vaccinating a subject, wherein the polypeptide comprises antigens of a pathogen that provoke a protective immune response against the pathogen.

In another aspect the invention relates to a use of the bacteria as defined above in the manufacture of a medicament for the site-specific delivery of a therapeutic polypeptide to the gastrointestinal tract of a subject. Preferably, the bacteria are used in the manufacture of a medicament for vaccinating a subject.

In yet another aspect, the invention relates to a use of the above defined host cells in a fermentation process, to the use of the above defined host cells as a probiotic or to the use of the above defined host cells as a live oral vaccine.

### Examples

### 1 Materials and methods

### 1.1 Bacterial strains and media

Bacterial strains and plasmids used in this study are listed in Table 1. *Escherichia coli* and *Bacillus subtilis* were grown in Luria (L)-broth-based medium at 37°C (25). *L. plantarum* and *Leuconostoc lactis* were grown in MRS broth (Difco Laboratories) at 37°C and 30°C, respectively. *Lactococcus lactis* was grown in M17 broth (Difco Laboratories) supplemented with 0.5% glucose at 30°C. *Streptococcus thermophilus* was grown in M17 broth supplemented with 0.5% of lactose at 42°C. *Carnobacterium piscicola* was grown in BHI medium at 30°C. If appropriate, the media contained chloramphenicol (10 µg/ml), rifampicin (50 µg/ml), or erythromycin (5 µg/ml).

**Table 1:**

| strains and plasmids. | | |
|---|---|---|
| Strain or plasmid | Relevant characteristic(s)^{a} | Reference |
| Strains | | |
| *E. coli* DH5α | Plasmid-free strain | (Hanahan, 1983) |
| *L. plantarum* WCFS1 | Single colony isolate of NCIMB8826 | Kleerebezem et al., 2003 |
| *L. plantarum* NC8 | Plasmid-free strain | (Aukrust and Blom, 1992) |
| *L. plantarum* NC8R | Rif^{r} derivative of NC8 | This application |
| *L. casei* LMG6904 | *L. casei* type strain (ATCC393) | Hansen PA and Lessel EF Lactobacillus casei (Orla-Jensen) comb. nov. Int. J. Syst. Bacteriol. 21: 69-71, 1971 |
| *L. helveticus* CNRZ 32 | Plasmid-free strain | (Khalid and Marth, 1990) |
| *L. lactis* MG1363 | Plasmid-free strain | (Gasson, 1983) |
| *Lc. lactis* NZ6091 | Plasmid-free strain | (David et al., 1989) |
| *S. thermophilus* ST11 | Plasmid-free strain | (Mollet et al., 1993) |
| *C. piscicola* LV17B | Plasmid-free strain | (Ahn and Stiles, 1990) |
| *B. subtilis* 168 | Plasmid-free strain | Kunst et al. 1995, Nature 390: 249-256. |

| Plasmids | | |
|---|---|---|
| pNZ124 | Cm^{r}, 2.8-kb pSH71 replicon | (Platteeuw et al., 1993) |
| pUC18Ery | pUC 18, Ery^{r} | van Kranenburg et al., 1997 |
| pWCSF101 | isolated from WCFS1 | This application |
| pWCSF102 | isolated from WCFS1 | This application |
| pWCSF103 | isolated from WCFS1 | This application |
| p701 | Cm^{r}, replicon from pWCSF103 | This application |
| p706 | Cm^{r}, replicon from pWCSF101 | This application |
| p707 | Cm^{r}, replicon from pWCSF102 | This application |
| p708 | pUC18Ery, *arsB* | This application |
| p709 | pUC18Ery, *cadB* | This application |
| p710 | pUC18Ery, *traA* | This application |

| | | |
|---|---|---|
| ^{a}Amp^{r}, ampicillin resistant; Ery^{r}, erythromicyn resistant; Tet^{r}, tetracyclin resistant, Cm^{r} chloramphenicol resistant. Strain WCFS1 was deposited under the Treaty of Budapest at the CBS, Baam, The Netherlands on 27 June 2003 and was given accession number CBS113118. | | |

### 1.2 Heavy-metal resistance

The sensitivity towards heavy metals of *L. plantarum* strains was tested by inoculating MRS with concentration series ranging from 0 to 10 mg/ml of arsenite (NaAsO₂), arsenate (Na₂HAsO₄.7H₂O), cadmium (CdSO₄.8/3H₂O), cobalt (CoCl₂), copper (CuCl₂.2H₂O), iron (FeSO₄.7H₂O), lead (Pb(NO₃)₂), mercury (HgCl₂), nickel (NiCl₂.6H₂O) or zinc (ZnCl₂) with 2% of an overnight culture of the *L. plantarum* strains and determining the turbidity of the culture at 600 nm after 16 h of incubation.

### 1.3 Conjugation

Conjugation was performed using filter matings. Cells from overnight cultures of the donor (2 ml) and recipient (200 µl) were mixed, collected on a sterile 0.45 µm filter in a plastic filter holder using a syringe, and washed with 25 ml of sterile H₂O. The filters were placed on MRS agar plates and incubated under anaerobic conditions at 37°C for 20 h. Subsequently, the cells were resuspended in 5 ml of ¼× Ringers solution. Dilutions were plated in triplicate on MRS containing 10 mg/ml sodium arsenate for determining the donor count and on MRS containing rifampicin and 10 mg/ml sodium arsenate for determining the transconjugant count. The plates were incubated under anaerobic conditions at 37°C for 2 days.

### 1.4 DNA isolation and manipulation

Isolation of *E. coli* plasmid DNA and standard recombinant DNA techniques were performed as described by Sambrook *et al.* (25). Small-scale isolation of plasmid DNA from the Gram-positive bacteria was performed using log-phase cultures as described previously (8). Large-scale plasmid DNA isolation for *L. plantarum* was performed by the method of Anderson and McKay (2). Electrotransformation was performed using a Gene Pulser apparatus (BioRad). Electrocompetent cells of *E. coli, L. plantarum, Lactococcus lactis* (8), *Leuconostoc lactis* (*23*), *Streptococcus thermophilus* (22), *Lactobacillus helveticus* (4), *Carnobacterium piscicola,* and *Bacillus subtilis,* were prepared as previously described.

### 1.5 Nucleotide sequence analysis

Sequence data were assembled and analysed using the PHRAP assembler (Ewing, B. & Green, P. (1998) *Genome Res.* 8: 186-194) and open reading frames were predicted using by using Genemark (Isono, K., McIninch, J. D. & Borodovsky, M. (1994) *DNA Res.* 1: 263-269) and Glimmer 2.0 (Delcher, A. L., Harmon, D., Kasif, S., White, O. & Salzberg, S. L. (1999) *Nucleic Acids Res.* 27: 4636-4641) trained on known *L. plantarum* genes. The SWALL and EMBL prokaryote libraries (version 3.2t05 May 1999) were screened for homologies using the Fasta3 WWW service at the European Bioinformatics Institute (EBI) (Pearson, 1990). The multiple sequence alignment was performed using the ClustalW WWW service at the EBI (Thompson *et al.,* 1994).

### 1.6 Construction of plasmids

To construct the cloning vectors based on the replicons of the endogenous L. *plantarum* plasmids, pWCFS101 and pWCFS102 were digested with *Xba*I and blunted. The replicon from pWCFS103 was obtained by PCR using the high-fidelity Pwo polymerase (Boehringer Mannheim). Cloning sites were introduced in the primers (underlined) with the sequences 5'-GCCGCGGTCGACAAGCCCCTATTCTTCTGTTT-3' (SEQ ID NO: 7) and

5'- GCCGCGCTCGAGTAAGCAAAGCCTGTATGTAA-3' (SEQ ID NO: 8).

The PCR product was digested with *Xho*I*-Sal*I*.* A fragment containing the multiple-cloning site and chloramphenicol resistance gene was cut from pNZ124 as a *Xho*I*-Sal*I fragment, blunted for pWCFS101 and pWCFS102, and ligated with the three replicons. For each ligation, one orientation of the resulting plasmids was used for further research and designated pR706 (from pWCFS101), pR707 (from pWCFS102), and pR701 (from pWCFS103).

### 1.7 Construction of single-crossover integrants

To construct single-crossover disruption mutants of *arsB, cadB,* and *traA,* respectively, internal gene fragments of approximately 0.4-0.5 kb were generated by PCR. *Kpn*I and *Bam*HI cloning sites were introduced in the primers (underlined)

5'-CCGCGCGGTACCGTTACTGGATCGTTTG-3' (SEQ ID NO: 9)and

5'-CCGCGCGGATCCACATTCTTAGGGCATAC-3' (arsB) (SEQ ID NO: 10),

5'-CCGCGCGGTACCATTCTTATACATCAACTGCAATCGA-3' (SEQ ID
NO: 11) and

5'-CCGCGCGGATCCAACAAACAGCGTTACGATTAGCTG-3' (cadB) (SEQ
ID NO: 12), and

5’-CCGCGCGGTACCAAAATGGGCGAGTGATCGGGAGA-3’ (SEQ ID NO:
13) and

5’-CCGCGCGGATCCCTTGATCGACAAATGATTTTTCGCT-3’ (traA) (SEQ
ID NO: 14).

The PCR fragments were cloned into pUC18Ery (28) and the resulting plasmids were designated pR708 (*arsB*), pR709 (*cadB*), and pR710 (*traA*), respectively. These plasmids were electroporated to *L. plantarum* WCFS1. Erythromycin-resistant colonies harboured pWCFS103 with single-crossover integrants, the integrity of which was checked by Southern blot hybridisation. For each knockout, a single-copy integrant was selected for further research.

### 1.8 Relative-copy-number determination

Plasmid copy-number was assessed using real-time PCR detection. For that purpose, plasmids pR701, pR706 and pR707 were transformed to *L. plantarum* strain PB100 that harbours a single copy of a chromosomally inserted erythromycin resistance gene and was obtained via a standard two-step homologous recombination strategy. This chromosomal marker was used as a chromosomal copy-number reference to which all plasmid copy numbers were compared. Real-time PCR amplification was performed using exponentially grown *L. plantarum* containing either pR701, pR706, or pR707 that were lysed. Cells were harvested from cultures (1 ml) by centrifugation, washed with water and pelleted. Pellets were lysed by microwave treatment (3 min., 800 W) and resuspended in 400 µl miliQ water. Appropriate solutions of these suspensions were used as template for PCR reactions. These reactions contained the primer pairs designed on the chromosomal marker *ery* (TM-ery-F99: 5'-TTCACCGAACACTAGGGTTGC-3', SEQ ID NO: 15, and TM-ery-R100: 5'-ATTCCGCTGGCAGCTTAAG-3', SEQ ID NO: 16) combined with the FAM reporter and TAMRA quencher dye containing *ery* probe (TM-ery-FAM: 5'-FAM-TGCACACTCAAGTCTCGATTCAGCA-TAMRA-3', SEQ ID NO: 17) and the primers designed for detection of the plasmid derived chloramphenicol resistance gene *cat* (TM-cat-F96: 5'-TCAAATACAGCTTTTAGAACTGG-3', SEQ ID NO: 18, and TM-cat-R97: 5'-ACCATCAAAAATTGTATAAAGTGGC-3', SEQ ID NO: 19) combined with the VIC reporter and TAMRA quencher dye containing *cat* probe (TM-cat-VIC98: 5'-**VIC**-GCGACGGAGAGTTAGGTTATTGGG-**TAMRA**-3', SEQ ID NO: 20). Reactions were performed with the TaqMan core reagent kit (Applied Biosystems, The Netherlands). The thresholds cycle number (Cₜ) was determined (Higuchi *et al.,* 1993) using the ABI Prism™ 7700 sequence detection system software and was used to calculate the relative gene copy number (Nᵣₑₗₐₜᵢᵥₑ) for each plasmid in relation to the chromosomal copy-number with the formula: Nᵣₑₗₐₜᵢᵥₑ= 2^{(Ct,cat-Ct,ery)}.

### 2 Results

### 2.1 Sequence analysis of pWCFS101, pWCFS102, and pWCFS103

The complete nucleotide sequences of the three plasmids found in *L. plantarum* WCFS1 were obtained by a shotgun approach. The average G+C contents were 39.5%, 34.3%, and 40.8% for pWCFS101, pWCFS102, and pWCFS103, respectively, compared to 44.5% for the complete genome of strain WCFS1 (18). All open reading frames larger than 40 amino acids were compared to the SWALL database (available at www.cmbi.kun.nl). The results are depicted in Table 2. Plasmids pWCFS101 and pWCFS102 are cryptic, while pWCFS103 contains several genes for heavy-metal resistance, NADH-oxidase activity, conjugation, and a complete transposon.

### 2.2 Replicons

The replicons of plasmids pWCFS101 and pWCFS102 share homology with known rolling-circle replicating plasmids. pWCFS101 has a pC194-type replicon (Khan, 1997) with a single replication gene *repA* encoding a 37 kDa protein that is very similar to the Rep proteins from *Lactobacillus hilgardii* plasmid pLAB1000 and *Lactobacillus plantarum* plasmid pLP1 (Bouia et al., 1989, Josson et al., 1990). Downstream of the *repA* gene at position 1798 begins a sequence (5'-TTCTTATCTTGATA-3') which is identical to the double-strand origin of pC194 (Gros et al., 1987). Plasmid R1162 carries a set of three-and-a-half 20-bp repeats that are essential for the expression of incompatibility and copy number control (21). Likewise, plasmid pLP1 contains a region with 13 contiguous direct repeats of 17 bp that are suggested to have a similar function (Bouia et al., 1989). pWCFS102 has a region with five-and-a-half direct repeats of 17 bp (5'-AGTGCGCATTATCATGT-3') that are identical to those of pLP1 and may serve the same function.

pWCFS102 has a pMV158-type replicon (Khan, 1997) and carries two replication genes. The *repA* gene encodes a 6 kDa protein that is homologous to copy-control proteins like RepC of pWV01 (Leenhouts et al., 1991). The *repB* gene encodes a 25 kDa protein that is highly homologous to the Rep proteins from *Lactobacillus helveticus* plasmid pLH2 and *Lactobacillus curvatus* plasmid pLC2 (Klein et al., 1993, Pridmore et al., 1994). A putative double-strand origin is formed by a sequence starting at position 2003, with high similarity to the double-strand origins of pWV01, pFX2 and pLC2 (Grohmann et al., 1998, Klein et al., 1993). These sequences are able to form a stem-loop structure and contain the nick site at which the Rep protein cleaves the DNA. The Rep binding region of pMV158-type replicons is composed of a set of two or three iterons, which are separated from the nick site by an intervening region of 13 to 91 bp (Khan, 1997). Unlike the nick regions, the Rep binding regions are not conserved in their nucleotide sequence (Khan, 1997). We could not detect any clear direct repeats in the region close to the putative nick site, nor elsewhere on pWCFS102.

The RepA protein of pWCFS101 and the RepB protein of pWCFS102 both contain the three conserved sequence motifs described by Ilyina and Koonin, one of which includes the putative DNA-linking Tyr residue (position 233) (Ilyina and Koonin, 1992).

Plasmid pWCFS103 has a replicon that is homologous to theta-replicons. The *repA* gene encodes a 12 kDa protein that shares homology with the C-terminal part of the replication proteins from the *Lactococcus lactis* plasmid pCI2000 and the *Lactobacillus helveticus* plasmids pLJ1 and pLH1 (Kearny et al., 2000, Takiguchi et al., 1989, Thompson et al., 1999). Compared to those replication proteins, RepA lacks approximately 260 amino acids at its N-terminus, the region that has strongest conservation and includes the helix-turn-helix motif that is considered to be involved in DNA binding (Thompson et al., 1999). The *repB* gene encodes a 30 kDa protein and is homologous to proposed plasmid copy control proteins from the *Enterococcus faecalis* plasmids pAD1 and pAM373, and the *Bacillus thuringiensis* plasmid pAW63 (De Boever et al., 2000, Weaver et al., 1993, Wilcks et al., 1999). Downstream of *repB* starts *orf4,* of which the start codon is overlapping with the *repB* stop codon. Therefore, it is likely that *orf4* is transcriptionally linked to *repB* and may be involved in replication. For pAD1 and pAW63 a similar small gene is found that partly overlaps *repB* (Weaver et al., 1993, Wilcks et al., 1999). In between the *repA* and *repB* genes is an iteron region with 11 and 10 copies of the sequence 5'-TGTATCCT-3' spaced by 37 nucleotides. Plasmid pAD1 also has a series of 25 8-bp direct repeats located in (Dougherty et al., 1998, Wang and Macrina, 1995), preceded by an TTAAG sequence is found upstream of *traA* and may function as the *oriT.*

To study the conjugal transfer of pWCFS103, *L. plantarum* WCFS1 and NC8R, a rifampicin-resistant derivative of NC8, were used in filter matings. Conjugants were selected on plates containing rifampicin to select for the recipient and sodium arsenate to select for plasmid pWCFS103 (see below). Typical frequencies of conjugation were between 4.4 10⁻⁶ and 3.1 10⁻⁷ per donor. Conjugation of the erythromycin-resistant derivatives of pWCFS103, pWCFS103::pR708, pWCFS103::pR709, and pWCFS103::pR710 resulted in similar frequencies of transfer. For pWCFS103::pR710 this was unexpected as this derivative contains a single-cross over integration in *mobA,* thought to be involved in conjugation by encoding the nicking enzyme. As MobA is a large protein, it cannot be ruled out that an additional start site downstream of the integration, still gives rise to a functional nicking enzyme.

### 2.6 Heavy metal resistance

Plasmid pWCFS103 contains two gene clusters that are predicted to be involved in arsenate/arsenite and cadmium resistance (Table 2). Bacterial arsenite transporters provide resistance to salts of the metalloids arsenic and antimonite (Nies, 1999). Arsenic mainly occurs as As(V) in arsenate and As (III) in arsenite. Arsenate is structurally related to phosphate and interferes with phosphate metabolism. It will be reduced to arsenite and exported (Nies, 1999). There are two types of arsenic resistance (*ars*) operons. One consists of three genes, *arsRBC,* coding for a regulator ArsR, the arsenate reductase ArsC and a secondary transporter ArsB that couples extrusion of anions to the transmembrane electrical potential (Driessen et al., 2000, Rosen, 1999). The other has two additional genes, *arsDA,* inserted in between *arsR* and *arsB* (Driessen et al., 2000, Rosen, 1999). ArsD is a second transcriptional regulator and ArsA an ATPase that associates with ArsB and converts it into a primary ATP-coupled arsenite transporter with improved resistance (Driessen et al., 2000). The pWCFS103 *ars* operon is such a high-resistance gene cluster, but lacks the arsenate reductase gene (*arsC*) and has two copies of the *arsD* regulator gene.

To test whether pWCFS103 confers to heavy metal resistance and if the *ars* and *cad* genes play a role in this, *L. plantarum* WCFS1, *L. plantarum* WCFS 1::pR708, *L. plantarum* WCFS1::pR709, and *L. plantarum* WCFS1::pR710 were incubated in media containing various concentrations of arsenite, arsenate, cadmium, cobalt, copper, iron, between *repA* and *repB,* which is predicted to function as the origin of replication (Weaver et al., 1993).

### 2.3 Host range

Based on the three replicons, three cloning vectors were constructed that contain the chloramphenicol resistance gene and multiple cloning site from pNZ124 (see materials and methods). For pR703 the 3.2-kb fragment with *repA, repB,* and *orf5* contained the complete replicon and could be stably maintained in *L. plantarum* NC8.

Plasmid stability was tested by sequential transfer in medium without antibiotic selection pressure in which over 80% of the colony-forming units still contained the plasmid after 400 generations. The host range of the three cloning vectors was determined by isolating plasmid DNA from *L. plantarum* NC8 and testing its ability to transform to various gram-positive organisms and *E. coli* (Table 3). The theta-type pR701 demonstrated a narrow host range and seemed restricted to *L. plantarum.* The rolling-circle-type pR706 and pR707 had a broader, but limited host range, as both were able to replicate in *Lb. helveticus, Lb. casei, Lc. lactis* and pR707 also in *C. piscicola* and *B. subtilis.*

### 2.4 Plasmid copy number

Plasmid copy number determinations were performed in triplicate and related to the chromosomal copy number. These experiments revealed that the relative copy-numbers of pR701, pR706 and pR707 were 4.7 (+/- 0.4), 11.9 (+/- 1.2), and 2.8 (+/-0.3), respectively. Therefore, these plasmids all generate a different gene dosage of genetic elements cloned on these vectors.

### 2.5 Mobilisation

Plasmid pWCFS103 carries a large gene cluster that has high homology with the mobilisation gene cluster of the *Lactococcus lactis* plasmid pMRC01. The *L. lactis* Tra region comprises an origin of transfer (*oriT*) sequence flanked by a gene cluster of 18 genes (*traA, orf6, orf7, traB, traC, traD, traE, traF, orf13, orf14, orf15, traJ, traK, orf18, traL, traI, orf21, ltrC*), and a gene cluster of two genes in the opposite orientation (*orf 4* and *orf3*) (Dougherty *et al.,* 1998). The pWCFS 103 Tra region runs from *orf37* (*orf3* in pMRC01) to *orf17* (*ltrC* in pMRC01) and is flanked by two transposase genes. Compared to pMRC01, it has an additional ORF, *orf20,* of unknown function in between *traL* and *traI.* Although there is no clear homology in the *oriT* region, the CGAAG sequence, conserved among several conjugative plasmids mercury, or nickel. For arsenite, and arsenate strain WCFS1::pR708 with *arsB* disrupted was more sensitive than strains WCFS1, WCFS1::pR709 (*cadB*) and WCFS1::pR710 (*traA*). At 500 µg arsenite per ml WCFS1::pR708 barely showed growth, while for the other strains this was at 5 mg arsenite per ml. A very limited effect on resistance towards iron, and mercury, was observed. At close-to-lethal concentrations the growth of WCFS 1::pR710 was slightly better than that of WCFS1::pR709 with *cadB* disrupted. No effect of pWCFS103 on cobalt, copper, iron, or nickel resistance was observed.

### References

1. **Ahn, C., and M. E. Stiles.** 1990. Plasmid-associated bacteriocin production by a strain of *Carnobacterium piscicola* from meat. Appl. Environ. Microbiol. **56**:2503-2510.
2. **Anderson, D. G., and L. L. McKay.** 1983. Simple and rapid method for isolating large plasmid DNA from lactic streptococci. Appl. Environ. Microbiol. **46:**549-552.
3. **Aukrust, T., and H. Blom.** 1992. Transformation of Lactobacillus strains used in meat and vegetable fermentations. Food Res. Int. **25**:253-261.
4. **Bhowmik, T., and J. L. Steele.** 1993. Development of an electroporation procedure for gene disruption in *Lactobacillus helveticus* CNRZ 32. J. Gen. Microbiol. **139:**1433-1439.
5. **Bouia, A., F. Bringel, L. Frey, B. Kammerer, A. Belarbi, A. Guyonvarch, and J. C. Hubert.** 1989. Structural organization of pLP1, a cryptic plasmid from *Lactobacillus plantarum* CCM 1904. Plasmid **22:**18 5-192.
6. **David, S., G. Simons, and W. M. De Vos.** 1989. Plasmid transformation by electroporation of *Leuconostoc paramesenteroides* and its use in molecular cloning. Appl. Environ. Microbiol. **55**:1483-1489.
7. **De Boever, E. H., D. B. Clewell, and C. M. Fraser.** 2000. *Enterococcus faecalis* conjugative plasmid pAM373: complete nucleotide sequence and genetic analyses of sex pheromone response. Mol. Microbiol. **37**:1327-1341.
8. **de Vos, W. M., P. Vos, H. de Haard, and I. Boerrigter.** 1989. Cloning and expression of the *Lactococcus lactis* subsp. *cremoris* SK11 gene encoding an extracellular serine proteinase. Gene **85**:169-176.
9. **Gasson, M. J.** 1983. Plasmid complements of *Streptococcus lactis* NCDO 712 and other lactic streptococci after protoplast-induced curing. J. Bacteriol. **154**:1-9.
10. **Grohmann, E., M. Moscoso, E. L. Zechner, G. del Solar, and M. Espinosa.** 1998. In vivo definition of the functional origin of leading strand replication on the lactococcal plasmid pFX2. Mol. Gen. Genet. **260**:38-47.
11. **Gros, M. F., H. te Riele, and S. D. Ehrlich.** 1987. Rolling circle replication of single-stranded DNA plasmid pC194. Embo J. **6**:3863-3869.
12. **Hanahan, D.** 1983. Studies on transformation of Escherichia coli with plasmids. J Mol Biol **166**:557-80.
13. **Ilyina, T. V., and E. V. Koonin.** 1992. Conserved sequence motifs in the initiator proteins for rolling circle DNA replication encoded by diverse replicons from eubacteria, eucaryotes and archaebacteria. Nucleic Acids Res. **20**:3279-3285.
14. **Josson, K., P. Soetaert, F. Michiels, H. Joos, and J. Mahillon.** 1990. *Lactobacillus hilgardii* plasmid pLAB 1000 consists of two functional cassettes commonly found in other gram-positive organisms. J. Bacteriol. **172**:3089-3099.
15. **Kearney, K., G. F. Fitzgerald, and J. F. Seegers.** 2000. Identification and characterization of an active plasmid partition mechanism for the novel *Lactococcus lactis* plasmid pCI2000. J. Bacteriol. **182**:30-37.
16. **Khalid, M. N., and E. H. Marth.** 1990. Purification and partial characterization of a prolyl-dipeptidyl aminopeptidase from Lactobacillus helveticus CNRZ 32. Appl. Environ. Microbiol. **56**:381-388.
17. **Khan, S. A.** 1997. Rolling-circle replication of bacterial plasmids. Microbiol. Mol. Biol. Rev. **61**:442-55.
18. **Kleerebezem, M., et al.** 2003. The complete nucleotide sequence of Lactobacillus plantarum WCFS1.. Proc. Natl. Acad. Sci. **100**:1990-5.
19. **Klein, J. R., C. Ulrich, and R. Plapp.** 1993. Characterization and sequence analysis of a small cryptic plasmid from *Lactobacillus curvatus* LTH683 and its use for construction of new Lactobacillus cloning vectors. Plasmid **30**:14-29.
20. **Leenhouts, K. J., B. Tolner, S. Bron, J. Kok, G. Venema, and J. F. Seegers.** 1991. Nucleotide sequence and characterization of the broad-host-range lactococcal plasmid pWVO1. Plasmid **26**:55-66.
21. **Lin, L. S., Y. J. Kim, and R. J. Meyer.** 1987. The 20 bp, directly repeated DNA sequence of broad host range plasmid R1162 exerts incompatibility *in vivo* and inhibits R1162 DNA replication *in vitro.* Mol. Gen. Genet. **208**:390-397.
22. **Mollet, B., J. Knol, B. Poolman, O. Marciset, and M. Delley.** 1993. Directed genomic integration, gene replacement, and integrative gene expression in *Streptococcus thermophilus.* J. Bacteriol. **175**:4315-4324.
23. **Platteeuw, C., G. Simons, and W. M. de Vos.** 1993. Use of the *Escherichia coli* b-glucuronidase (*gusA*) gene as a reporter for analyzing of promoters in lactic acid bacteria. Appl. Environ. Microbiol. **60**:587-593.
24. **Pridmore, D., T. Stefanova, and B. Mollet.** 1994. Cryptic plasmids from *Lactobacillus helveticus* and their evolutionary relationship. FEMS Microbiol. Lett. **124**:301-305.
25. **Sambrook, J., E. F. Fritsch, and T. Maniatis.** 1989. Molecular cloning; A laboratory manual, 2nd ed. Cold Spring Harbor Laboratory, New York.
26. **Takiguchi, R., H. Hashiba, K. Aoyama, and S. Ishii.** 1989. Complete nucleotide sequence and characterization of a cryptic plasmid from *Lactobacillus helveticus* subsp. *jugurti.* Appl. Environ. Microbiol. **55**:1653-1655.
27. **Thompson, J. K., S. Foley, K. J. McConville, C. Nicholson, M. A. Collins, and R. D. Pridmore.** 1999. Complete sequence of plasmid pLH1 from *Lactobacillus helveticus* ATCC15009: analysis reveals the presence of regions homologous to other native plasmids from the host strain. Plasmid **42**:221-235.
28. **van Kranenburg, R., J. D. Marugg, S. van, II, N. J. Willem, and W. M. de Vos.** 1997. Molecular characterization of the plasmid-encoded *eps* gene cluster essential for exopolysaccharide biosynthesis in *Lactococcus lactis.* Mol. Microbiol. **24**:387-397.
29. **Weaver, K. E., D. B. Clewell, and F. An.** 1993. Identification, characterization, and nucleotide sequence of a region of *Enterococcus faecalis* pheromone-responsive plasmid pAD1 capable of autonomous replication. J. Bacteriol. **175**:1900-1909.
30. **Wilcks, A., L. Smidt, O. A. Okstad, A. B. Kolsto, J. Mahillon, and L. Andrup.** 1999. Replication mechanism and sequence analysis of the replicon of pAW63, a conjugative plasmid from *Bacillus thuringiensis.* J. Bacteriol. **181:**3193-3200.

**Table 2:**

| Putative genes and their products, deduced from the nucleotide sequence of pWCFS101, pWCFS102 and pWCFS103. | | | | | | |
|---|---|---|---|---|---|---|
| CDS | start | stop ^{a} | No. of amino acids | Best hit (organism) | % Identity (in # amino acids overlap) | Proposed function of gene product |
| **pWCFS101:** *rep* | 163 | 1122 | 319 | Rep protein pLAB 1000 (*Lactobacillus hilgardii*) | 71 (314) | replication protein |
| *orf2* | 1406 | 1260 C | 48 | no hits | | hypothetical protein |
| *orf3* | 1627 | 1809 | 60 | no hits | | hypothetical protein |
| | | | | | | |
| **pWCFS102:** *repB* | 102 | 761 | 219 | RepB-like protein pLH2 (*Lactobacillus helveticus*) | 88 (219) | replication protein |
| *orf2* | 1393 | 953 C | 146 | no hits | | hypothetical protein |
| *orj3* | 1889 | 1686 C | 67 | no hits | | hypothetical protein |
| *repA* | 2242 | 35 | 52 | CopA pPSC22 (*Lactobacillus plantarum*) | 60 (50) | copy-number control protein |
| | | | | | | |
| **pWCFS103:** *orf1* | 51 | 35839 C | 93 | DinJ (*E. coli*) | 30(80) | DNA-damage-inducible protein |
| *repA* | 610 | 302 C | 102 | RepA-like protein pSAK1 (*Lactobacillus sakei*) | 53 (76)^{c} | replication protein |
| *orf3* | 925 | 1257 C | 110 | no hits | | hypothetical protein |
| *repB* | 2061 | 2861 | 266 | RepB pAD1 (*Enterococcus faecalis*) | 39 (276) | copy-number control protein |
| *orf5* | 2863 | 3213 | 116 | hypothetical protein (*Lactobacillus sakei*) | 33 (113) | hypothetical protein |
| *orf6* | 3344 | 3586 | 80 | no hits | | hypothetical protein |
| *tnpR1* | 4370 | 3816 C | 184 | Resolvase-like protein pMD136 (*Pediococcus pentosus*) | 83 (184) | putative resolvase |
| *orf8* | 4649 | 4855 | 68 | no hits | | hypothetical protein |
| *orf9* | 5641 | 5378 C | 87 | no hits | | hypothetical protein |
| *arsR* | 5713 | 6072 | 119 | ArsR-like protein pLI100 (*Listeria inocua*) | 48 (119) | regulator of arsenical resistance |
| *arsD1* | 6059 | 6421 | 120 | ArsD-like protein pLI100 (*Listeria inocua*) | 42 (123) | operon repressor of arsenical resistance |
| *arsA* | 6505 | 8235 | 576 | ArsA-like protein pLI100 (*Listeria inocua*) | 65 (568) | operon arsenical pump-driving ATPase |
| *arsB* | 8294 | 9589 | 431 | ArsB-like protein (*Listeria monocytogenes*) | 73 (427) | arsenical pump membrane protein |
| *orf14* | 9606 | 9935 | 109 | no hits | | hypothetical protein |
| *arsD2* | 9958 | 10257 | 99 | ArsD-like protein pLI100 (*Listeria inocua)* | 43 (95) | repressor of arsenical resistance |
| *nox* | 10281 | 11681 | 466 | hypothetical protein pLI100 (*Listeria inocua*) | 36 (450) | operon putative NADH oxidase |
| *cadC* | 12031 | 12399 | 122 | transcriptional regulator ArsR-family (*Chlorobium tepidum*) | 37 (97) | putative positive regulator of cadmium resistance |
| *cadD* | 12401 | 13015 | 204 | CadD pUB101 (*Staphylococcus aureus*) | 50 (205) | putative cadmium resistance protein |
| *orf19* | 14131 | 13100 C | 344 | transposase (*Lactobacillus delbruekii*) | 41 (338) | putative transposase |
| *orf20* | 14404 | 14240 C | 54 | LtrC pMRC01 (*Lactococcus lactis*) | 61 (49)^{b} | hypothetical protein |
| *orf21* | 14623 | 14408 C | 71 | hypothetical 8.0 kDa protein pMRC01 (*Lactococcus lactis*) | 57 (70) | hypothetical protein |
| *traI* | 16881 | 14746 C | 711 | TrsI pMRC01 (*Lactococcus lactis*) | 60 (722) | DNA topoisomerase |
| *orf23* | 17298 | 16888 C | 136 | no hits | | hypothetical protein |
| *traL* | 18170 | 17313 C | 285 | TrsL pMRC01 (*Lactococcus lactis*) | 44 (277) | conjugation protein |
| *orf25* | 18565 | 18176 C | 129 | hypothetical 14.7 kDa protein pMRC01 (*Lactococcus lactis*) | 32 (126) | hypothetical protein |
| *traK* | 20076 | 18565 C | 503 | TraK pMRC01 (*Lactococcus lactis)* | 76 (503) | conjugation protein |
| *traJ* | 20548 | 20078 C | 156 | TrsJ pMRC01 (*Lactococcus lactis*) | 48 (150) | conjugation protein |
| *orf28* | 20917 | 20549 C | 122 | hypothetical protein pMRC01 (*Lactococcus* | 42 (118) | hypothetical protein |
| | | | | *lactis*) | | |
| *orf29* | 21521 | 20904 C | 205 | hypothetical 23.3 kDa protein pMRC01 (*Lactococcus lactis*) | 48 (204) | hypothetical protein |
| *orf30* | 22690 | 21536 C | 384 | hypothetical protein pMRC01 (*Lactococcus lactis*) | 54 (395) | hypothetical protein |
| *traF* | 24109 | 22691 C | 472 | TrsF pMRC01 (*Lactococcus lactis*) | 50 (471) | conjugation protein |
| *traE* | 26120 | 24102 C | 672 | TrsE pMRC01 (*Lactococcus lactis*) | 82 (671) | conjugation protein |
| *traD* | 26791 | 26132 C | 219 | TrsD pMRC01 (*Lactococcus lactis*) | 80 (219) | conjugation protein |
| *traC* | 27122 | 27760 C | 120 | TrsC pMRC01 (*Lactococcus lactis*) | 73 (115) | conjugation protein |
| *traB* | 27478 | 27143 C | 111 | TrsB pMRC01 (*Lactococcus lactis*) | 72 (107) | conjugation protein |
| *orf36* | 28094 | 27480 C | 204 | hypothetical 23.2 kDa protein pMRC01 (*Lactococcus lactis*) | 51 (201) | hypothetical protein |
| *orf37* | 28471 | 28133 C | 112 | hypothetical 13.1 kDa protein pMRC01 (*Lactococcus lactis*) | 46 (90) | hypothetical protein |
| *traA* | 30584 | 28524 C | 686 | TraA pMRC01 (*Lactococcus lactis*) | 49 (677) | nickase |
| *orf39* | 30856 | 31065 | 69 | hypothetical 10.9 kDa protein pMRC01 (*Lactococcus lactis*) | 56 (66) | hypothetical protein |
| *orf40* | 31088 | 31366 | 92 | hypothetical 10.6 kDa protein pMRC01 (*Lactococcus lactis*) | 50 (88) | hypothetical protein |
| *orf41* | 31356 | 31670 C | 104 | No hits | | hypothetical protein |
| *tnpR2* | 32466 | 31880 C | 195 | TnpR transposon Tn551 (*Staphylococcus aureus*) | 40 (181) | putative resolvase |
| *tnpA* | 32645 | 35638 | 997 | transposase pAM373 (*Enterococcus faecalis*) | 64 (987) | putative transposase |

| | | | | | | |
|---|---|---|---|---|---|---|
| ^{*a*} C, complementary sequence | | | | | | |
| ^{*b*} Homologous to N-terminal part of protein | | | | | | |
| ^{*c*} Homologous to C-terminal part of protein | | | | | | |

**Table 3:**

| Host range of *L. plantarum* WCFS1 cloning vectors | | | |
|---|---|---|---|
| Host | pR706 | pR707 | pR701 |
| *L. plantarum* NC8 | + | + | + |
| *L. helveticus* CNRZ 32 | + | + | - |
| *L. casei* LMG6904 | + | + | - |
| *Lc. lactis* NZ6091 | + | + | - |
| *L. lactis* MG1363 | - | - | - |
| *S. thermophilus* ST11 | - | - | - |
| *C. piscicola* LV17B | - | + | - |
| *B. subtilis* 168 | - | + | - |
| *E. coli* DH5α | - | - | - |

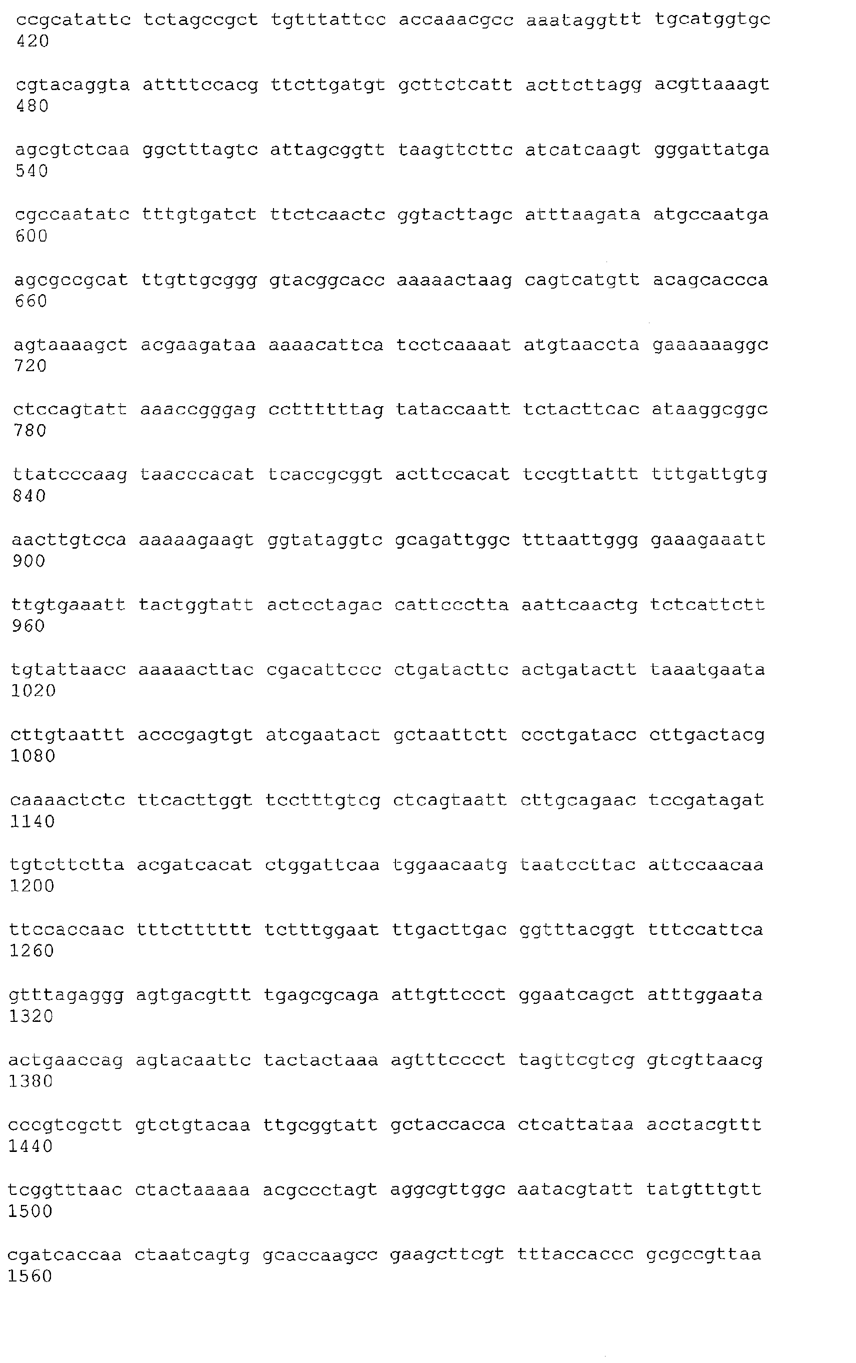

## Claims

1. An isolated nucleic acid molecule comprising a replicon derived from pWCFS101, pWCFS102 or pWCFS103.

2. A nucleic acid molecule according to claim 1, whereby the replicon comprises all the cis elements necessary for replication of the plasmid in a *Lactobacillus* host cell.

3. A nucleic acid molecule according to claims 1 or 2, whereby the replicon:
(a) comprises the nucleotide sequence of SEQ ID NO: 1;
(b) comprises the nucleotide sequence of SEQ ID NO: 2; or,
(c) comprises the nucleotide sequence of nucleotide positions 260 - 3410 in SEQ ID NO: 3.

4. A nucleic acid molecule according to any one of claims 1 - 3, whereby the nucleic acid molecule is a plasmid.

5. A vector comprising a plasmid as defined in claim 4 and a gene that confers to a host cell containing the vector a phenotype that allows to discriminate a host cell containing the vector from a host cell that does not contain the vector.

6. A vector according to claim 5, whereby the gene is a selectable marker gene.

7. A vector according to any one of claim 5 - 6, whereby the vector further comprises a nucleotide sequence capable of replication in *Escherichia coli.*

8. A vector according to any one of claim 5 - 7, whereby the vector further comprises a promoter that, when the vector is present in a suitable host cell, is capable of driving transcription of a nucleotide sequence operably linked to the promoter.

9. A vector according to any one of claim 5 - 8, whereby the vector further comprises a nucleotide sequence of interest.

10. A vector according to claim 9, whereby the nucleotide sequence of interest encodes a polypeptide and whereby the nucleotide sequence is operably linked to the promoter that is capable of driving transcription of a nucleotide sequence in a suitable host cell.

11. A bacterial host cell comprising a nucleic acid molecule or a vector as defined in any one of claims 1 - 10.

12. A bacterial host cell according to claim 11, whereby the host cell is a bacterium that belongs to a genus selected from the group consisting of *Lactobacillus, Lactococcus, Leuconostoc, Carnobacterium, Bifidobacterium,* and *Bacillus.*

13. A bacterial host cell according to claim 16, whereby the host cell is a bacterium that belongs to a species *L. plantarum,* and whereby all nucleotide sequences that are present in the nucleic acid molecule or a vector are homologous to *L. plantarum.*

14. A method of transforming a bacterial host cell, the method comprising introducing into the host cell a nucleic acid molecule or a vector as defined in any one of claims 1 - 10.

15. A method for producing a fermentation product, wherein the method comprises culturing a bacterial host cell as defined in any one of claims 11 - 14.

16. A method for producing a polypeptide of interest, wherein the method comprises culturing a bacterial host cell as defined in claims 11 - 14, under conditions conducive to the expression of the polypeptide.

17. Use of a bacterial host cell as defined in claim 11 - 14 for the manufacture of a composition for site-specific delivery of a polypeptide to the gastrointestinal tract of a subject, whereby composition is administered to the gastrointestinal tract of the subject.

18. A use according to claim 17 for the manufacture of a composition for vaccination, whereby the polypeptide comprises an antigen of a pathogen that provokes a protective immune response against the pathogen.

19. Use of a host cell as defined in any one of claims 11 - 14 in a fermentation process.

20. Use of a host cell as defined in any one of claims 11 - 14 as a probiotic.

21. Use of a host cell as defined in any one of claims 11 - 14 as a live oral vaccine.


SEQUENCE LISTING

<110>  Stichting Top-instituut Voedselwetenshappen

<120>  Vectors based on novel plasmids derived from Lactobacillus
plantarum

<130>  P205056EP

<140>  P205056EP

<141>  2003-05-28

<160>  20

<170>  PatentIn version 3.1

<210>  1

<211>  1917

<212>  DNA

<213>  Lactobacillus plantarum

<400>  1
aattcctagt ttggcgaccc ggaacacgtg agttaatctt gaatattcgt atttactaga
60

catagtttaa agcttgagtt agtaagcgtc aagcccttag ttttagtaaa tacataaaag
120

attagctctt ctcacgtggt tggatgagag gagcttttta gtttggctga tagaaaagtt
180

ttagttgatc gatcgcagtc gggaaaagta cgaccatggc gagaacataa gttagagaat
240

ttacagtatg gtgattattt acaaatgttg cactataaga aatcccatcg agttaaagag
300

tgtggtgaag tattacgttt tgtggaagat aaaaatggtc acaaaaaatt ggctcagact
360

tggttttgcc attcccgttt gtgtccgtta tgtaattggc ggcggtcaat gaaacaatct
420

aaccagttaa ctcaaatttt gacagaagca gttaaacagc gaaaaacggg tcggttcttg
480

tttttaacgt tgacggtgaa gaatactaca ggggatttgt tgaagagtga gttacggcag
540

atgggacgag ccattgcaaa gatctttcag tataaaaaag tggctaaaaa tttgttgggt
600

tatgtacgtt caactgaggt taccattaat cacgaagcag atcagccgat gtatcaccac
660

catatgcatg ttttgctttt tatgaaatcg agttatttta caggaactga taattatatt
720

tcacaagcgg aatggaccgg gtattggcaa cgagcgatga aattagatta tgtgccggtt
780

gtgaatgttg aagcggttaa accgaatgtg aaacgccaga aaaattcctt gctggctagt
840

gcccaagaaa cggctaaata tcaggtaaag tccaaagata ttttaactaa taatcaagaa
900

caagatctac aagtaattga tgatttggaa caagctttgg ctggttcccg ccaaattagt
960

tatggtgggt tactgaaaga aattcgcaaa cagttgcaat tggaagacgt tgaaaatggt
1020

gatttgatta atacggatag tgatgatcaa aaggttgatc aagtggtgcg tgagattgtt
1080

gctaaatggg attatcagcg taagaattat tttgtatggt aattttagaa aaaggacgga
1140

tgactgatcc gtcctttttg tttttgatgc agtgttttct gtttctagaa tgacttcaat
1200

ttatttggag tgtaagtgcg cattatcatg tagtgcgcat tatcatgtag tgcgcattat
1260

catgtagtgc gcattatcat gtagtgcgta ttatcatgta gtgcgcactt acacacaaca
1320

tgaagttgtg ttgtgctaaa cccatcaaaa cctgcatcag atttcgcgtt gctcaaacgc
1380

aactgacttg cgtcagtttg gaacattcaa aaataaataa gttcagtcgc tagctccttc
1440

gaactttttt atttttgaac gttaatttta aaggctctta tttgcgttct aagcgatttt
1500

agctaacagt gatccatcta actgtctgtc aacggtaaat cgaattaaag ggacttactg
1560

ctttagcagt tagtcccttt ttgaggcttt aaggagttga ctgactaacc aaacccagac
1620

actgaattgg ctgaaagcca aagttcccta aactttgctt tcctgcctca cggcgagtgc
1680

ggggtgagtt tgagcgggag ctcccgctca attatgcggt caagctggca cagcttggac
1740

tgggttcggg gcgtcagcgc ccgagggctt agaaactggt cgattttgac cagtttcttc
1800

ttatcttgat actattagaa acaacgtcat tttaaaaaac cgggataaac ccttgacaca
1860

actgggctta ggcgtattat gagtttataa aatgaataaa gaaaaaaccc acgtgag
1917

<210> 2

<211> 2365

<212> DNA

<213> Lactobacillus plantarum

<400> 2
   attcaagaaa ggaatcagag caaaaaaata agtgagaact cattcccttc aagaaatacg
   60
   agttctcact aatgtttgtt ctgtcaaggc gattataaca aatggcaaaa gaaaaggcga
   120
   gatacttcac ttttttactt tatccagaat cgataccaca agattggcaa acaaaacttg
   180
   aaactttagg cgttccaatt gcaattagcc cgcttcatga taaagatttg agcagtgttg
   240
   aggggcaaaa gtataaaaaa gcccattatc acgttattta tatctctaaa aatcctgtca
   300
   cagcagaaag cgttcgcttg aaaatcaagc gtgctttagg cgataagagt gttgctaaag
   360
   tacaaattgt tgtccagagc atggaaaata tgtatttgta tctgacacat gaatctaaag
   420
   acgctattgc taaaaataag cacaagtaca gcaagcatga cattgctgta ctgaacaatt
   480
   ttgatattga tcgctatatt acgcttgatg ttgaagataa agacgacatg ctgaatgatg
   540
   tttgcgattt gattgatgac cataatttgg cgaatatgcg tgaactgaga cgctttttaa
   600
   aagctcatgg ttcagaatat ggcatgcccg gtattaaagt cgttaattcg gttttacgtg
   660
   ctcatactgg actgataagg ctgtatttcg atgctgttta tcaggaacgc aagtatggca
   720
   gaggcgatat aaacaaagag actggcgaga tacaagatta attagcgaat ggaaagtagg
   780
   tgttcgattt gagcaccttt tttgttgtcg gctagccgac ttctgataca agtctttagc
   840
   acaataacac aatcaacttt agttgttgtg tgtaagtgcg cccttaggaa tataatttga
   900
   atatatttca gattttcaat ctgactgcta ctatcatcaa gataagaaat aactaatttg
   960
   tgtcaaaaat tttctctaaa tctgtttcat caacattttc ttcaatttcc aaatctctat
   1020
   acatctcaac aaaaatattc actaattttt cattagcaag cccgtcatca accgatgaat
   1080
   ttgacttata gctataaaaa tttttataac ttttaataac atctggcgaa tcaaaaaaaa
   1140
   cagcaggaac actattaaac gctctcttaa attcagtgct agaaaaatca tatcttgaac
   1200
   caatcaattg aactaaaact tcatatttct tcatccaaat tcttcgttca tgttcctgaa
   1260
   ttgctaaggt attttggaaa ttttcattat ccgattctaa ttgtaattga aattttttac
   1320
   tccttttgtc attaaaaaat gagtaaaaca aagtaatagt cacacctacc aaacctaaaa
   1380
   taccacctac caaattaatt cctcctaaat ttttatttat attttatttt acgttatttt
   1440
   aagacgattt tagtattttt taataaaaat tatttataaa tcaaaaagta ctcttaaaac
   1500
   acagatataa gcaattaaga aaataaaaat ctcaaaaaac aaaatctcta ttttttaaaa
   1560
   atcgctatag ttcttgtggt gactgaaaaa acgagaatta atttgaaatt ggtgtttttt
   1620
   gatatgaaaa taaagtttag tcctcttttt gttttgaata gttctagaac atcatatttt
   1680
   gcgttctaag cgattttagc taacagtgag ctatttaact gtctgtcaac ggtaaatcga
   1740
   cttagagggg cttggagagc cttacaggcg atattagccc ctcttggagg ctttaaggag
   1800
   ttgatagact gtacgaaacc taaagcctga tgagcaggaa acaagccctt gtttccccga
   1860
   gcccagtggc ggcgaacgat agcggtcaag ctggctcagc ttggtctggg tttggggcga
   1920
   cagcgcccaa gggttttaaa aagacgtttc agcgattttt gctgatccgt cttttttggt
   1980
   ttgtgttgta aataaatttt tagcgctgag gggggcaagg aagcaaattt tgcgaccgta
   2040
   ctacgacccc ccttttaagt gccgagtgcc aaaattgaat ttttcgggac cttatcccta
   2100
   ctctcccaag gactacagcg attaaaaaaa aatcaacaaa tcgccaacaa aattggcaaa
   2160
   aaaacgtgtt gatgtaaaat aacttgacaa aaaaagcgaa taaaagtaaa atttgtttac
   2220
   aatatttaag aggagaatat tatggctgaa aataaaaaaa gagtttcgat tgggttgacg
   2280
   gataaacagt atgaattagt tcaaaaattg gctaaagaaa aaggcttttc gaagtctgca
   2340
   atatttgttt tagctttaga agaat
   2365

<210> 3

<211> 36069

<212> DNA

<213> Lactobacillus plantarum

<400> 3
caaatcttta tcaatcttga cttggacccg tttcttttcc ttaactgcca tgattgtctc
60

atctcctgtc ttttgctact accattatac taccttttga gtaccttatc aatagataaa
120

gccctggcct caaatcactt gagcttggcg ttgatctaaa ggctgaattt ttgagctggg
180

tagctctggc actcatttaa aagcccctat tcttctgttt aagccattta aatctagctt
240

aagcataaag aggttagtaa gtcttaaaac cgcttataaa ggattttgca gccttttagg
300

actagtagta caacccactg ttatcgttgt cggccgactt ttcttcttcg ggtgtttgg
360

tggtcgcgct caccattcac cacccagccg tacagatgac cttgttgtgg tatagccaga
1620

cttattgaag tcgcactggc aaacgtgtcc ccttggattt agaccccagc ttgtccccta
1680

gggctttaaa aatcgcaccg gccatgatat aatagtcagt aaatgagttg acttttatgt
1740

ggcgctcacc gattcccgtc ggtgggtgtt tttttatgtt ctattttatg taaccaatat
1800

tactcgttgt ttggtgataa atcaacattt ctttttatgt atccttgtat ccttgtatcc
1860

ttgtatattc atatccttgt atctaagtat ccttgtatct aagtatctaa gtatattagg
1920

atacttgcag tcaatcttaa tttaacggct tttgagaaga aatgtatcct tgtatccttg
1980

tatccttgta tctttgtatg ttatcatcct tgtatccaag tatccttgta tattaggata
2040

cttaattgga ggtcagtata atgggtgaag taattacttt tgggaatttt aaaggtggca
2100

ccggtaaaac aacaaatgcc acgcttgcat gcctggcact agctcgtgct ggtaaaaaaa
2160

cgctattaat tgatttcgat cctcaagcaa acgctacaga tatttacttt aaaaccgcgg
2220

ctaatttagg ccacgatgat attgaattta atcaaacctt gcttgcatca atccaagaag
2280

aggacttatc aagatcgttg gttactttgg ataaaaacat tgactttatt ccatcaagcg
2340

cagatttttc attgtaccca cggattatgg aaaaaaagtt taaaaataat tataaagata
2400

gagttacata ttttagcaag ctacttgctt cactaaaaga aaaatatgat tttgtggtat
2460

ttgatttgcc accgactatt tccttaattt ctgatagcgc cttatacgct tcagattggg
2520

ttctgattat ccttcaaact caagagcaca gtcttcaggg tgccgaatcg tttctaaaat
2580

atatacagga acaggtaatc gatgaatacg aagcacctag tttgaattta ctcggtatac
2640

ttcctgtgct attaaagaat ggggcgcctg ttgatcacag cacattagaa gtagcagaag
2700

aagaatttgg gaaagaaaac atgttaaaaa ccctaattcg taatatggaa agaatcaaaa
2760

gatattcaat cagaggtgta tttctaaaag atcagttcga taaaaaaata attagattat
2820

acgattctgt ggcacaagaa attattgaaa gggcggacta gcatggtgga acttttacac
2880

aatcctaact caaataaaag caaaataatt cctagaaaat cggcgcccca gccccaaaaa
2940

gaaatttcca tatcttctct aaacgaatca aacaaaacga agtctaaaca aatagatggt
3000

gttacttttg atactacgct cagaattgac aatcatctaa aaaactttat gaaagctatg
3060

gtaattttag ggtatagctc aactcaacaa aacggattag cccaattaca aaaaactttt
3120

tttgaatcgt taactgagtc tgaacaaaat acgcttacga ctcaaataca gactttggaa
3180

accggcgatg ctaacaaagt aaaaagtaaa taatcacgtg ctctcagacg acacgttcac
3240

cttttagatg tatacttgtc cgtttaaatt aagcagaatg cacacaaaaa agtcctctat
3300

tatgcggaat agagggcttt tctgaaatca ttacatacag gctttgctta ggagcgttgc
3360

ttgtgtccat ttaggacata cacaatcata taacaatcaa tgctgtcact taacttactc
3420

aaaaaagcca cccacctcgg agtggctttt tggttgtgtg gtagattgta aaatttttct
3480

tgtttctgcc caattgattc aattcagtaa ctatgacaac accaccttaa caaatcataa
3540

tttggataat agtatgttcc atttgaaagc taagccttat aattagcata gggttaatag
3600

ctctaatcaa aatacccaca aaagtcctgc tcaactaaca attgagtagg ccttttgtag
3660

tacaaattaa tccttatcac aactaacctc aatagcacaa caggtataaa tagtgcctaa
3720

aatccctaaa taaatttcta tcacaactgt ttaataaagc ggcaatcccc gttgtacgtg
3780

attaattaaa cattcaattc ttaaaacacc tatttttaaa tcgacttaaa ccttcgctgt
3840

tgcgtgcgtt tactaatccc cgtctttcgt tcaatcatct tataagtcat gccttgtttt
3900

cgcaactcat aagcaaatct gatctgttca tcagaatacg ttttcggtcg cccttcccga
3960

aacaacggat catgttgctt ggcatacaat ttaccttctt gtgtgcgcgt gacaatcata
4020

tcgcgttcaa actgcgcaaa ggcgctaaat attgtaaaga ctaattggcc agtcggcgta
4080

ttgtcaatta agcccatatt caaaatgttg actttgacat tttctttaaa caactcttgg
4140

ataatggcta aggcctcgcg cgtgttccgt gcaaaccgat ccaacttagt gacaatcaaa
4200

gtatcgcctg tttttagaac gcacaacagt ttttgaaact ccggtcgttc agtagttgtg
4260

ccagtaaact tttcctggaa gatttttctt gctcctgcca attttagtaa ctcaatttga
4320

tttgctaatt tttgatcagt ggtactgacc cgcgcatagc catatttcat ctttttatct
4380

ccttatttat gtcattaagt aatgacacgg ttctaaccct ttaattatac agcatcaaaa
4440

aagaggccac aactgttgag ttgtgtcgct ttttaaggtg cgcccggcat gggcagtaac
4500

taggcggtgt aagtccgctg tgagccgtag tggccggtat cacgagtcga agacaagggc
4560

gtctatcgtg aggtagaatc tgaaggaagt cctagacgaa acatcgaatc gatgaacaag
4620

aagtggctaa aaggctggaa actgtgggat gagattacct aacaaatcga agtccctgac
4680

cactcgaaac cgtcacagta aagccaacgg tttgatgcga aagctactgc tcttacccgg
4740

ggaggtctgt catggtagtt ctctcaagct ggaagttatt tctggcagga acaggctcaa
4800

tagtaatgtt gagttgtcca tgacagaagt cagccgaggc catagtagcc agtaatggtg
4860

aggggctgaa caacaataat tttgattcat cctggaggtg gaaacaatgc gacaatcgca
4920

gaaaacagaa aataaagctg actacctgcg gagagtaacc ctggaagctc actataaaaa
4980

atgactagat attgtaaaaa agcagcggga taatatgaga tttaagtagc caaaagcctg
5040

ataaaacggg cttttttgtt tgccgttgat ttgaaagttt agatatcttt ttctcccaat
5100

tttgtataga aatttggccg attgagcaat aataagcaat gattatcaaa ttatggtacc
5160

aaaattggta ccgcaaaaag gccgccggga catatggact tccttttatg gatttgaata
5220

agttattttc aaaacgaaat atcgatccaa gtttgaagtt ggttaatcta tcggcttaaa
5280

aaactaatca tggaagaatg tgattattca gtatgatcta aaatagatgc tgctatttta
5340

tttcaaaata aagcttgacg cttacataga tatgggtcta tattatagat atagatgcac
5400

gtctatgttt acaaaatacg atgtttcttg cggtgatcga agcaagttga gttgaggaaa
5460

tgcagttgcg gagtttgcaa ctaagttccc taaaatcacc acgaaagcgg tcataatcat
5520

tcattttggc aacactgatt agcaagaagt atctgcttgt tgaatatttt ctgctaaaca
5580

ggattggctt tctgttgttg attaagccaa aaccaaaagt agcaagttca gtctgtatca
5640

ctgtaacatc aatgtttatt attagcgtat ttaactaata gattacctta attatggaat
5700

aggagcttac aaatggacta tcaaaagtat gttcaaatgc tgaaagcaat ggccgatcca
5760

aatcgcttaa aaattattga tctgctttcc tgtggttctt tgtgtgcttg tgatatttta
5820

gagcacttcg acttttcaca gccgacttta tcccaccata tgaaagtttt gcaaaatgct
5880

ggtattgttg cggcacgcaa ggaaggtaag tggcagcatt atacgttacg ccccgatttt
5940

atgctgacct ttaagcagga tacggcgcaa ttattatcaa gtgatgatca ctgtatttgc
6000

cacgatgacg gctgtgttga ttgtgaagaa tgtgcttcag gaaaggttgt tatgacaaat
6060

gaaaaaaatt gaattatttg aaccagcaat gtgctgctcg actggggttt gtggtccgtc
6120

cgttgatcaa aatctcttaa tgatcacgtc tgcctttgat gctttacaag gtgttaaaac
6180

gattgaagct gaccgttaca atttaagtaa taatcctgat gtttttagtg aacgagccga
6240

tattttagcg gccatcaagg atgatgctga cgctgtttta ccgatcaccg tggttgacgg
6300

ccagatcgtt aaaacgggcg cgtacccaac gatcgatgaa ttatctgact atactgggct
6360

agttttcgtg ccagctgagc aaacaggcgg ttgctgtggc ggtgatggtg gctgttgcta
6420

aattttacta aaggtaagag atggcgcggc catttctttt gaattatata tagataaata
6480

tctatgtgac tgaggaggat atcaatgcag gactatcaac cacaaaacgc taatttaact
6540

cattatttgt tttttaccgg taaaggtggc gttggtaaaa caacgacggc cagtgcaacc
6600

gccattaatt tggctgatgc tggtaatcaa gtgatgctcg tttcaactga cccagctagt
6660

aatttacagg atgttttcaa tacagcatta accaataaac cgcaagcaat caatggtgta
6720

tcagggttgt ttgccgccaa ttttgatcct gtgaccgcag ctggtgagta tcgggaaagt
6780

gttgttggcc cttatcgcgg cgtattgccg gatgctgctg ttaaaaatat ggaagaacag
6840

ctatccggtt cctgtacggt cgagatcgcg gcattcaatg aatttgccaa ctttttaact
6900

gatccggcgg ttgaccagcg ctttgattat attatttttg acacggcgcc aactggtcat
6960

gccttgcgca tgctacagct accgtcagcg tggaataatt atttagatga aaatgatcgc
7020

ggtgcttctt gcttaggcca attagccggt atgggtgata aaaaagcaat ttatgcaaaa
7080

gcggtagcaa cgttaagcaa tggggacttg accacattaa tgttggtgac gcggccacaa
7140

aaagcctcat tacttgaagc agcacgagca gcacaagaac tagccgcgat cggcatgact
7200

aatcagcagt tgatcattaa tggcacgctt aaaacaccga cggatcacgc ttcgcaggct
7260

atttttgcgc aacaacaggc tgatctacaa caaatgccgg ctgtattaaa aaaatttgct
7320

cagtatgaag tgccgttacg cgcctacaac gttaccggct taaataaatt acacctggtg
7380

ttacaggcaa cacaacccgc attagctacc tatccgccgc tgaccaataa ctatcccaac
7440

ttagatacga tcgttgctga tctgatcaag acggataaaa agattatttt taccatgggc
7500

aaagggggcg tcggtaaaac aacggttgcg gtgcagatca cgcagaaatt agtggcccaa
7560

cataaaaccg ttcatctagc caccactgat ccggccgatc atctagattt ctttaaaaca
7620

gctgatccgg cagttacgat cagccatatt gacgagcagc aggtattgaa agattatcaa
7680

gctgaagtac tggcaacggc tcgccaaacc atgaaagcgg ctgacgttga ttatgtcgcc
7740

gaagatctcc gttcaccatg tacgcaagaa attgcggttt tccgcgcttt tgctaatatt
7800

gtggcgcaaa acgacagtga cgtggtggtg atcgatacgg cgccaactgg gcacacgttg
7860

ttactattag attccactca aagctatgca caggaagtta agcgcacagc cggtgacgtg
7920

ccacaagcaa ttattgatct attgccacgg ttgcaagatc cccaacaaac cgagatcgtg
7980

atggtgactt taccggaaac aacgccagtc tacgaatcaa tgcggctcaa tgaagattta
8040

aaccgggcgc aaattgccca tacatggtgg ttggtcaatc aaagcatgct ggctacacaa
8100

accacgcatc cgtgtttaca agcacgggca caaaatgaag ttgaatggat cgaaaaagtc
8160

aaagacgttt cggccaatca ttttgcagtg gaacagtggc aaccagattt tgaacaaacg
8220

ctattaacga tttaaataaa gatatcaatt gatcagggta gcgataccct gattttggta
8280

gttgggagga attatgcaag tcttttttgc tgtttttata tttttactca cgttattatt
8340

cgtcatttgg caacctaagg ggctatcaat aggctggacc gcaattggtg ggtcagtact
8400

tgcactgcta tttcgggtcg tcacatttaa agatgtgggt accgttactg ggatcgtttg
8460

gaacgccact ttgtcgtttg ttgccattat tttgatctca ttgattttag atgagatcgg
8520

ctttttcgag tgggccgctt tacatatggc acgactggct aaaggcaatg gtgtacgtat
8580

gttcattttg atcgttattt taggtgcgat cgtggcggca ctttttgcca acgatggggc
8640

ggcattgatt ttaacgccga tcgttctggc aatggtgcga gccctacatt ttgatgaaaa
8700

aaaggttttc ccatttatta ttgccagtgg ctttattgcc gatgcgactt cgttaccatt
8760

agttgttagt aatctagtca atattgtttc cgctgacttt ttcggcatta cattttccga
8820

gtatgcccta agaatgtgga tccctgatct tttttcactg atcgccagca tcagtatctt
8880

atatttgtat ttccgcaagg ccttacccaa acattatgat gcagatcagg ttgctgaacc
8940

aaaatctgca atcaaagatc aacgattatt taagttctct tgggtcgtgt tagcggcgtt
9000

attgatcggt tattttagca gtagttttct aaacattccc gtttcgttta ttgcacttac
9060

gattgccgca attttcttgt tggttggtca acaaagtcat cacgttgata ctaaggcagt
9120

cattaaagga gcaccgtgga acatcgtctt cttttcaatc ggaatgtatg tggtggttta
9180

tggcctacaa aatgttggct tgacaacatt acttgctggt gttatcgcca aaatggcaac
9240

tggtagccgg tttgtggcga ccatgggaat gggttatttg gcagcgttcc tgtcatcagc
9300

gatgaataat atgccaacgg taatgatcaa tgctttatcg atcaagggcg ctaatgtatc
9360

agggttaatg caccaaacgt tagtttatgc aaatattatt ggttctgatc taggaccaaa
9420

gatcacacca atcggttcgc tggcgacgtt agtgtggtta catgttttgg cgcaaaaaaa
9480

cgtaaaaatt gcttggggta cttattttaa aattgggatc acgatcacga ttccagtcct
9540

attcattaca ttatgtggtt tatggttatc gctaagtctt tttggttgag aaaggatgac
9600

ttgctatgat gcaaactgag tatgcacaac aagattcatt agctaataga ttaaacatag
9660

tcgatgcttt agtgaaacag gccacgaaag ctgaaataga atcaatttct gctgtttacc
9720

tgattcaagc caaccaagaa attaatttgc ttgaattaaa atacagtagt gaattaacaa
9780

tacaacagaa cttgtataag gtaaaaatag cctttttgca aataaatcac ttgattttaa
9840

ataatctagc ggtcagctat tttcgtgatt atacacaaat agaaaaggaa tttgaacatt
9900

ttcatcgtct aatatgcgag gtgaaagacg attaaaattg aaatatttga agaggcgatg
9960

tgttgtgcaa ctggtgtttg tggtcccagt gtaaagaaag aattgattca gactaccgca
10020

attcaacgtc atgttaatgc agatggtgaa aatataatca tacgcagaaa tcttacacaa
10080

aatccggacg cctttgttcg taatcatcaa ataaatgacc tattaaaaaa atatggaatc
10140

actgtattac caattactat ggttaacggt ataattttta aacaaagaga ttatccaacc
10200

ttccaagagt tttcagatta tctacatcag gatttaagtc gtgcatttgt tcgctaacaa
10260

cggtgaaagg aattaattaa atggcaaatg aaacggaaaa attattaatt atcggcggca
10320

gtgacgccgg aatttcggcg gccttaaagg ctaaagaact taaacctgaa ttaaaagttc
10380

agatattatt agcagatgag taccccaatt tatcaatttg tggaatacct tatgcagtca
10440

gcggagaagt gccggattgg cacttgctag ctcatcgtaa tttacaagaa ttgacttcga
10500

ctggtattga atttcaaatg aacatgattg cagataaaat tgaaccgcaa cgacatgaag
10560

tcgtagcaca tgcgcttaca ggcgaattaa aaacttatca ttatgaccac ttggttgtag
10620

caacaggggc tagaccaaaa ctatcgggta ttaaaggcgt ggacctggcg caagctcaac
10680

agcaagatag taaagttcgt attttgcata caatggcaga ttactttgcg attgaaagaa
10740

atatagctac agatagtgtt cggaaggtcg caattgttgg gtctggttat atcggaatcg
10800

aaatggcgga ggctttaaga aaacggcatt tagacgtcac tatttttcaa cgaggttcag
10860

aaatactttc cacggttgat gctgatttag gccgaattat tcatcaaaag ttgtctgcta
10920

atgccgtcaa tgttattact ggtttaaccg tttcagaaat taatgaaact ggtagcaacg
10980

tggaagttgt tggcttaaat gagcatcaaa agacacaatc ttctacgtat gatttagtct
11040

taatcgttgt tggtgttcaa ccaaataatg agctactggt caaggtaggt gctgaaactg
11100

gaattgccgg ggccatcgag gttgatcaat atatgcagac ctcattacct gatatatggg
11160

cggccggcga tttggtggaa acaagacatc atttattagg tgaaacctac ttgccattag
11220

gtacgactgc acataaacaa ggaagaattg ctggttttaa tattgctgga attcaacgtg
11280

cctttaaagg tagcctgggg acacaagttt taaaggcatt tgatttagtt gttgtacgga
11340

cagggttatt gacaagtgag gctatccagg cagggtttaa tcctttcacg gtaacaactg
11400

atgttgatga ccataaggct tattttcccg gtgctcaaaa aattaagatc agaattactg
11460

gtgatcaaaa aagtggccgg cttttaggcg tgcagttaat tggtcattac ggtagtgaag
11520

ttgctaaacg cagtgatatt tttgctacgg caatttttaa tgatatgaca gtagccgaaa
11580

ttagcgacct agacttatcc tattcaccac cagttggatc accttgggat gcagtacaaa
11640

tcgcagcaca gaactgggaa caacagaatg cgaaacgcta aggaattaat ttataactga
11700

aggaacaact tcacttcatt attaattaag ctttctacta tctgttttta tatgattacc
11760

agattactct gaggacaatg attggcggct atttatattg aaagaattaa aatggcgcaa
11820

aatgatagta acgaaacttt ggcatcttct ggtagattaa attaaaacta agacctgtat
11880

cttagtttta atttaactag tgaatatgaa tagtggttaa tgtcagatag agtggctata
11940

ggagctactc tattttttga ttcaaattaa attggggaca acactattac atgatataat
12000

gtaatagatt aataaacgag gagtgatcta atgatacaag tagactgttg tccgggtaaa
12060

ccagatttgg ctgatcgggc attgttaaat gagaatcaag ccgctgacat tgctttactt
12120

tttaaagtac tcgcaaataa tacgcggcta agaattttac attgcttaac gcgtgaacca
12180

gatatggccg ttagtgagat tgctacatca ctaaatatga aggtccaagc catctcaaac
12240

cagttacaac gtctagtaga tcaaaagata gttaaagcga agcgtaatgg caacttcatt
12300

gaatatcaga tcattgatga atgtacggca attttattag aacgggcatg gtgtttagct
12360

gaagatgccg gaaaaatcaa tgctggtggg ggaaaataaa atgatcagga caattataac
12420

gagtattatt ctttatacat caactgcaat cgacttatta gttattttaa tgttactttt
12480

ttcaaaatat cgttcaacaa agcatcgccg acaaatttat attggtcagt tcttaggctc
12540

atatacgctc attggaatta gtttgttttt tgcatttatt ctccattacg tacccgccaa
12600

atggttacta ggttttttag ggttgattcc cattgccttt ggaatcaaat atatatttag
12660

caaagaagat gaagccgcag aagttgatca gaaaattgaa gcgcgtaaaa ataagaattt
12720

aattgcaacg gtggctttaa ttacggtagc ttcttgtggt gcggataata ttggcctttt
12780

tgttccttac ttcgtatcat tatcactggt acagctaatc gtaacgctgt ttgtttttac
12840

catttgtgtt tattttttag ttttccttgg tgataagttc tcacaagtga attttgtaaa
12900

gaactttctt gatcgttttg gtgattggat catggccatt atttatattg gtttaggcat
12960

catgatcatt cttgaaagtg acactattag gcatattatg cagctattat tttaacccaa
13020

attgcaagaa caaattagcc acacgataaa tcctattaaa tcagtatttt cattaatatt
13080

catattttat gggcttaact tagccggcgg aagcagcgga gaaaagctct ttgggtgttt
13140

gatagccagt ttgccggcgc ggtaaattgt ttagctttga ctcaactgct tggatatcat
13200

gaggacccaa aatatccatg gatagaccct tgggtacatc gcggcggatc attcgattat
13260

gcgcttcgtt agtcccacgc tcagaggaac gatatggatg ggcataataa aggtcagcga
13320

caccggtaag tacagcgcca acttccgaga actcagcgcc attatcggca gtaactgact
13380

tcatgattga tccgtattct tgacagatct tggccagctc atagttgacc gaatcggcat
13440

cacgaccatc gatcaaacgt agaatttggc agcgggattg acgctcgatc agcgttaaga
13500

tcacactctc ttgaccgttg cgcttgccca ccacggtgtc caattcgaaa tggccaaact
13560

cttggcgctg gtcgatactt ttaggccgtt catcaatact ccgtccggcc agacgcttgt
13620

gcttgatcga gtgatgatgt ttggcacggt ggcgcgtttt ttcgagtaga tcaagattac
13680

ggacttcaag taactgggca tcaatatagt ggtatagcgt cttggtcgag accatctcct
13740

caggttgata taagccttca agtttggccc gtcctactgt tgcatcaggc gaccaaccat
13800

cttgacgcaa atgagtcgta aaatagtcga taaaatcagc gacaccgacg agtttcaaag
13860

gtcgatgaca gtgggctcga ttagcttcgt acttagcttg tgctgtttct ggtgagtata
13920

cagcgtaata ttggcgttga ccattcactt tttttacttg gtcgatctcg ccgcggcaca
13980

attcattatt gattgtttga tgacaaacat taagcgctct ggcaattgca cgattggaat
14040

agccttggct gtgcagcgta gcaattctgc ctcgttcaaa cgaagttagg tgctgacctt
14100

tttttctgac tgtggtattc tgttcttgca tcaagacaat aacctctttc attgtttgtg
14160

taggaacatc aatgataaca gagatttgtc ttggtgtttt ttattttatc aattagctaa
14220

aagtatttgg ctaacttgat tataaaacgc gcgattcaat gtttctggcg ctataatgat
14280

gaaatttagc cagggtatta agatactgtt taaatcggtc actatcggtt aattttagga
14340

tttgttgttc agcctgggcg actaattgtg ctttccaggc tttaacgtcg gctttactcg
14400

gcattgtcta cccctcctca tcttcaaaaa tatcattcaa tataggggct acgttgattt
14460

gaaccggggt cgtgccataa agggcaacgt agccatcggc taatttttgc caagccactt
14520

gataataaga cccatttcca gtaatttctt ttaaatattt gtaggttcct ttagtctgca
14580

aaaccggtaa ttcactccca atgactgtta attcgttatc catatttatg atctcctttg
14640

cgttgctaat ctcacccgtt gtttaggttt acttttaggg ttgtaaaagt gaacctaaat
14700

gacgggttcg ccaaccggaa cgtagtgtag ggcggtaggc aggttctaat caaaatcaaa
14760

acttaattta tggccgtcaa gctttaactt ggcgtcaaac gactttcctt ttttgctctt
14820

gaaccccttt agtttgctgg tttccccctt agtgattaag gctttgattg cagttttacc
14880

gagggtttta ctgctccatt tcttgggcag ggtaaagtct tcgccttgct taggctttac
14940

aatgtaaaac ttctgtttat ttaaaacggt agcttgcggt gtttctaaga agacctcggc
15000

ggctttttgg gcttgctgct gatgatcgat ttgttgctta atggctgcac tgccagttaa
15060

ttgcgtggga acatcagcaa tcaactttgc cacgaacttt ttgatttggt tcaaaaagtt
15120

atctggggta cgttcttcgg tactgatttg ttgtaacgct tgctcccatt tcgctgtcat
15180

ctctgggcta gttaacaagg gttcgagttc gaccgcttta cataaagtaa ttccggcttc
15240

actgacgtgt aacttgttct tttcagtgac gagatagccg cgcttcttta acacttccag
15300

cacattggcc cgggtcgcac ttgtcccaat gccttgcaca tctttgagaa tcgcctgggc
15360

ttcttcatca tcaagcgttt taccggcggt tttcattgcc gtaatcaggg taccttcggt
15420

aaagggtacc ggtggtgtcg tttctttttg cggcgtttgc aggtttgctt ggacttgatc
15480

gccttggtga acgatcggta aagttgctgc tgcttgctgg tcggctgtgt gatcatcaaa
15540

taatgcttgc caaccttgct tcgttggaac cttaccggtt gctttaaaat tggcgtcacc
15600

aacttgagtg ataatggtgg tttcttcgta ctcatacgga tcagcaaaca tcgctaacgt
15660

cgttcttaaa accaagtcat agacttgttg ctgtaattta ggtaagctgg ctagtttctc
15720

tttagtcggc acaactttag tcatgataat agcgtagtgt tcttcaacct tttcccatt
15780

gacgtaacgt ttattcgggg tggtattggt taaagcgact tgcttagaga ctaaccccag
15840

atacttcgtc agattagcca ctaaatactc aaattcttca tcagtgatat aagcacaatc
15900

ggttcgggga tagctaagga acttggcttc gtataaactc tgaatggccg ctaaggtttg
15960

gctggcactg gcatgatacc gtttattcat cgcactttgc agactggata gcgagaatag
16020

ttgggaactg gcactctttt tagcctgctt ttgcacgtcc ttgattaaac cgtcctgtga
16080

tcctttctga acgtgtttag cttgcatgaa tgtcattagc tctgtttcat ctttaaatcg
16140

ctgatagggg tctaattttg cgacgaattt ttgctgattg gctaaaattt ccgcatttag
16200

ttcaaaatag ggttccggct taaagttttt gattgtctgg tctctttgat agaccatata
16260

taaggttggc gtctgtactc gaccaatcga atacaccccg cgtaccccgt tttgtcttaa
16320

taacaaagta tataaaggac tgccattcat accaattaac cagtcactaa tttgacgggt
16380

ttgggcttct ttataagcca aatagtcttt gtgccaatca ccaagatttt taaagccggt
16440

aataatcgcg tctttttcta ggctattcaa ccaaaggcgc ttaatcgttt tcgacttaac
16500

attaatatgc gcttggttca tgatcgacca cgcgatattt gacccttctc gaccactatc
16560

ggtggccaca ataatggtat cggctttcgt taacaggtct ttaacgatct tgaattgcgc
16620

tcttttacta gttgacactt caaacttgta tttgtcgggg aaaatcggta aattagataa
16680

ggcccattgc tggtgtttct gatcatattt atccggtgtg gctagttcaa ctaaatgtcc
16740

gagaccatac gtcacaagcg tattttcggg caaaacaggg tcactaaccg tatagtaacc
16800

ctgttttttt gtgctctttt gaaaggcttg gacgtatgaa cgggcttgac tgggtttttc
16860

agctaagata acagtggtca tgttcgttca ctccttgtga ttaagcttct gttctaattc
16920

tgttttgcct tgttcagatg cttttaacca agtttcacgg cacaattctt gttgctggtc
16980

tatcgattgt tgtttcaaat attgtcggta gcgattaatg gccttaatat ccgcatacgc
17040

tgaccagcga aataataatc caccaaatat taagataaac gccatcagtg ctgtaccaac
17100

aaaatcggtc ttatcagaag taaacagctc atgatacaag tctattaaca ttagatatag
17160

gcccattaaa tagaagataa tactggcaaa actatcgaaa tagtaagaca gttttgtgcg
17220

ccttggcggt atcgtaaagg gatcagtttg catgtccttt ttaatttttt taagtaacca
17280

gtgccagatt tttttcatga cgtgaacctc ccttaacggt tacgaaaata attttgaaac
17340

attctcatgg cacccatacc agcactctta gtcacaccgg ttgaagccca gaaatcttta
17400

acggcgtcag gtgttttgac aaccagaaag ccaaagccaa ttgaagcaat aaacgcaata
17460

aacccgtatt tagccatatt agtaaaacac catactgata atgacataca aagcacttgg
17520

ctaataatgg aggcattgta ataaatatac ttcttccacc acgtaccact gtaatcaaag
17580

ctttcagtcg ccatgctcat tgcagctaaa ggaattgtca ggttatacca agccatatca
17640

gccacaaaca cacacatttt aataaagaac acgacggtta cgacagtgaa gattagtaga
17700

aacaaaatct gtaagggtag tgaaatgcca acatctttta acccaggaac cttattcata
17760

ccggtaattg atttagcagt aaacttctga tcagaactaa acatatactt tagcaggggc
17820

aaggtaattg aaccttgaat aaagccctgt aagaacacga atactgggat tacggccgct
17880

gatttaatcc cgtccatgat gatattagcc caagtgacat cggtgctttc gtccgcctct
17940

tttagaattg tcatcacaat acgcccaagg acaaccacga ctactaacga tgtcgcaaag
18000

gacaggaaga tggcatacca cgttttaaca attggcaaac tttgattggc ttggtcaata
18060

atggccttgc agatgttgaa taaaccgtcc agcaaaccag ataatgccca ttcaaaaaat
18120

ttagcaatcg ctccctgaac taatcctgaa atgtcaccta aaacaaacat atctatcact
18180

tccttttttg caattcaaaa ctccctaatc cgttatctgc tttagcagac ttattaattg
18240

ctttagcctt ataaccatcg ctaatttgag ttaattcaaa cttttgattt ttctcatcat
18300

cagtatctac ttttaaatat tctttaccgg aagtattgct gactgaataa gtggcattta
18360

cattcatcaa agggctttca ataatcattg ttttttattt gaattgggtt ttgtagcttt
18420

gacctttttg gttgagatac catttgttac tttgcaaatc ttgtgctata gacttcccac
18480

aagcggttag accaacagca atcagtaaca ccgtcatcac ggcgattata tatttccgat
18540

attttttcat taatatgtcc tccattatcc aagcattttc ttatgtctag actctaattc
18600

ttgatcgata ttatcctgca atttgtcttc ttcgtcatcg gttgtttgat cgtctttttt
18660

ggcttggtgc tgtgctttaa ctgtctgatt ccacttatct accttattct taaagtgggt
18720

ttcctggctg gcttctggtt ggccttgata ctcattctgt gacaagttaa ccaaatgatc
18780

agcccctgga aatagtttaa attggaacgc ttttgtagct ttgactggtc gggcattact
18840

aaagattaat aagctctgat cttcaggcat acgcatgatt tcatcaggtg tcatcagcga
18900

acggctcgta tagctgtaac tatcgctctt actgtggctc tcttccttac tgtgactagt
18960

actttcactt cccgtttcca cttttacggt cgatttacct aacaaatcac taaagtattt
19020

agcagttacg tcattagcgg cattcaagca aattttaacc gcatgattac ctaagatact
19080

ttcggcttta tccttgccat acaaagcttg gagctgggtt aaggtctgac aaatggtcgt
19140

cacgccaatg ccgtaccccc gacacgtcgc taggaactcc tcatacttgg gaaacttccc
19200

taaattgaca aattcatcaa ggataaagtc gacttggtgg ggcaatttag cgtgatgatc
19260

ggcggctaat ttgtataatt catcaaataa ttgtgagaaa aacagattga tgaagctttc
19320

ataggtatta tccatgaccg gaataatcac atacattact actttggctt tgccaatctc
19380

ttttaaatca aagtctgaaa agctggtaaa atttgccact tctttgtcaa cgaacttcga
19440

aatggtcgcc aacaggcttt caataatgct ggctttcatt tctcctttag cctttttgaa
19500

acctaactca taagcgcgtc tcgctggatc agtcatagtt agatcaagaa ataaggtgtc
19560

taacggacta tctgcacctt tttcctcggc ttccacatcg ttttcttgca acacttgggt
19620

cacacccgcc aaattccgtt gttctggtga ccggtggttc atgacaaaca ggattaaagc
19680

cttcaaaagc tgtctttggg tactaaacca cacatctttt ttgccttcgg aattttcact
19740

ctggacgatc ttggtggcga cggtttcagc ttgaatatcc cgttgaatat aatcaaaagg
19800

attgtagcga tcgctgtgcg ccatgttggc aaagttgacg acatgtactt gataaccctg
19860

ggctagtttg ataccggccg tcttttcgta taattcccct tttggatcgg tcacgacaat
19920

gctgttctcc gtttcattaa acaggttggt aatgacgact gactgggttt tatatgatcc
19980

aggacccccg accacaaaga tattccggtt tggtttggtg ctgttattct ggtaaacaat
20040

ccgtttatcc acgatcccta aaattgtccc attcatgtta atctccccct ttcgctgact
20100

tttctcgatc cactaatgat ttgagaactt cggccttggt actcttgtta aatgctgtgg
20160

tgaggtcttt ggcattgata ctaaaataat ggtcactcaa ttcttttaga tcaccccagg
20220

tcgcactccc gtgagtttca gtatctgcag tttcccaagc tttgtgcttg ctgttgcgct
20280

tcaaagcaaa atagatggtg tataggatca cggcgacact gacaataatt aataccgggt
20340

tcttttggcc taaatataag ttgtaataat gccaggggtg taaagctaat tgttttaata
20400

cggttggcat atgatccgca aaggttagct tataacggga caaggccatg acagctccag
20460

ctaagatctc cgctaaatac aagccaatga ttagcgttag tagatatggc cagctgactt
20520

taacgctgtt taataatttg tctttcatct aatcacctac tatctgttta attctttgtt
20580

ggttggtgcc ggtgtaccgt tgcttaccat ggatcaaggt tggaactgac gttaattgat
20640

attgctgaat gtagtgccga ttctgcgttt gattcatgtt aataaaaaca acgttatggc
20700

taatgacgtt gtgccaatag attcggtgaa aaatagcttg gcaatccggg caatcatcgc
20760

gatagaagaa caacactttt ttagggtgct tagctaaagt cacgattggt cgctgttttt
20820

ctacgtggtt gacatattga tgactggcaa aaccactaac ggctaaaacc accacgatta
20880

ccgctagggc cttaccaacc cgcttaatta aggtcactgg ctttaaagct actacctaag
20940

ttttggattg cggtgattcg atccgtggtg gtatcgaaag tcacctgata aagtaccgtc
21000

gccttttgcc agtcgttgtc gccactctta ctttcataac taatgacggc caagcccttc
21060

atgttttgcc cctgcttact ttgcgtatac aggttcagtt gatttaactt agctgaaaca
21120

ctattgctat caccatatgt gcccttattt gaaaagtatt gctggtacaa cttgtcggaa
21180

atcaagcctt tcacgccatc tttacgctga ccgtaggtct tgggcttgaa gttattcatg
21240

acctcaaaga acttggtgac caccgtatta aagctcagtt cagcctgact tttgtcttta
21300

ttattttggt aggtcttata actgtcaatt tgagcgctca ataactgctt ttgttgatta
21360

gttttggtta attgccggct aacttgtcgc ttctgttgtt gtaagcgact gatctgttgt
21420

tgtgcctgct taatttggct atgctgatag gcattcgccc ctaatgacat aactaagatc
21480

aagctaccaa tcgcaatact taaaactacg ctacgcttca tttgtttgtc cctccttact
21540

tcttgattgg tcgggctaat gtcacatcac taccacttag taagctataa aaagcggtgc
21600

caaatgtgct ttcgttaacc ttgtcgccga caccaccttg ctcaatgatc ttggtggctt
21660

tgccttgcca cttatctagc agaatggcgg tatgcccgtt gttgccggcc ccggcgcctt
21720

gattaacaat cacaatatcg ccggccttcg cgtcattttc actgatctgt ttcagatatt
21780

gatgactgcc tttggcgtca cttgccatcg taccggtaaa ccagcccatg ttggccggtg
21840

ccttgtaacc agctttatta agcgctaacc agacaaagcc tgaacaatcg gttccaccgg
21900

ttttgttagg attctttaaa tcgctaccta aatcagggct agggtgggtt tgaacatagt
21960

agaaatcgcc tttcatactt tccgcatttt tgacgatact accgccggaa tagcttgggt
22020

cactatcaca acctagttcg gtcagatcgc cggaactcgc attgtctaag gtgtttccgg
22080

ccactggatc actcgcaccc aacacggaat accagtgatc ggcatatccg tatctttggc
22140

ttagatacca ttgttccggg ttcttactca taccctcata atccattagc caggctttcg
22200

tcgctttgtg cacgtccgta tatttaaaga tctgcttgtt tgacgcatag taacgactgt
22260

tcaattcttg gtcgagataa tccaactgta ccccgagatt ggtcgctggt ttgttttcct
22320

tgtgggctaa gtcttccagc ttatcctttc gaccgcctaa ccactgggct aagcccgtgg
22380

ccccggagct tgaattaacc gaattgggat caaggcgact ttctagttgt aagaccccta
22440

agatcccggc ggccgcttgt ggggtggcgc catacttctg tttccagtgc gcatagatat
22500

ttttggcatt ttctgtcatg ctcttactat ccaattgcgt ctctgtggtg gttgagttat
22560

cacaactgtt ttcttgtagc tgaccagtaa tcgcggcgat cagcaagacg attagcagaa
22620

tggcaccgcc cacaatataa gcaatcaact tccacttctt tttcttatat tcaaacgaca
22680

atacctgcat ttaatcaccc actttgtgct tggcctgatc aacttgtttt tgcgctgccg
22740

attggtcgct ctgcgcattc tttagatcag ccttagccga atccaccttc ttgtctttat
22800

ccttatcttt actgccatca atcttgttgg cgtcatctaa cttcttttgg gcgtcgctaa
22860

cttcgttctt agcagagttg agacttaata gcgctttgag atactgggct ttagggctgt
22920

tcaccccagc taatttggtt tggacggtct gggcgtcttg cgtatcgtta tgggctagga
22980

aggcttgtcc cattctcaat aaaacatcag cgttcgtcgt aaaggataat tggttattga
23040

gcgtattggt atcataatta acaattgctt tttcaagttt caactggtct tttgcttgc
23100

tcgtcgcctt agttggtaac tgccaattag ccacttgacc gctatctttg ttggcataag
23160

ccgcattaac aaccttgttc aactgacttg ggtcaacctt taaagcttgc tggtactggc
23220

catgctttaa aaaggcactg acttgcttag ttgaaggata gccggcccgt ttcatatctt
23280

tttgcgcttc cgaccacttc tgatcattta aagcggtttg aatttgaccg ctatatttta
23340

atcgtgtgac ctgggcttgg ttctggttag ctaagctttg atactttcgt tcgtcacttt
23400

tatggactaa gccgacggcc aaaacggcca caatcgccac cccggccata ctcaaccata
23460

aacgttgttt ggctttctgc tgggccgttt caacttcctg ccattcgtga tagctcacaa
23520

agtcctcaat gccgttaacc acttccgtta actcatttaa cgacgtagct ttagctactt
23580

gttgtaaata gtcgtccggg tgtttggtca aggcttcttg gggattgctt agtagccgtt
23640

catagggcgt ccccgtcaag caaaacagaa tcagtgcttt gtatttggct aaattgctat
23700

gtttgtcatc ataaggcata agttcattgg cccggtaggc gtaccaaaca tgttgcatgg
23760

ggtaatacca taggttggca gggttcaacg caatgtgata ctggctatcg ttaaccacat
23820

gttgcgccat gatttccttg gcaatcgctg accggattgc tttattttca tgtggcaagt
23880

cctttaatga tctgacctta tccggcaagg tataagttag aacgaccttc tggtcttgct
23940

caaccacgtt aaccaactgc aagaacttgg aatcagcttg ttttaattcg tttagctcac
24000

tcaactggtc gtatcgaaac tgattgtgat tgagttcgag aattaacttg ttcccctgtc
24060

gcttgaacgt gccgacctcg atatttaata gctggtttag tttactcatt taaagcccct
24120

ccttcctcct cggtagtggt caagtcgtca atttcactgg gagttaagac cacgcgcttt
24180

tgatagtccg gttgcccggc tgtctcacgg tgatattttt cttggtaagc ttctgggtca
24240

attagccgta gttcttcttc ggtcaactcg acacgcataa aggcgcgttg gctaccatag
24300

atcatcaggg cttcaccttg tctgcgacgc tctagtaact tcttttcttt atccgagaac
24360

gtcatacgca atttggtaat cacatcatca acgcctttac cgtcaaaacc aaagaatact
24420

ttggtgtagg aattcccaat aatggcttcc ccaatgttct gcccagtatc tgttgttcct
24480

tcgatcacat cttgaatttg ctgcgttccg gcaatggccc cggcattgta ttttctaaaa
24540

cgtttgtatg cttgatggaa aaaggtggcg gccgctttat gcaaggtcaa aaagtgaaat
24600

tcatcgacaa acaatttctt gcggctatgc cgatctttgg tgatttcgtc ccacaaatac
24660

tggaaggtat tcaaataagc cgctgattgg acttcctgtt cactttgtag tggttttaga
24720

tcaaaagaaa tgattttccc ttttaggtta acgttggtat gcccgtcaaa taagctgtta
24780

gaaccatggg tatagctgcc taagatatag taaaaatctt tgacccgatt aaatttgtct
24840

tcatctctat cagccagttt ctccaattcg tcataaacat tggataaggt cggccactga
24900

tcgtctttaa tctcttttaa gcggctgtat ttcaaaacac cactgttgac gtaggcctgt
24960

ttaaccacat catcaagaat tgccagctca acttgggtaa tcccggtttt aaggacttca
25020

aagaaccctt ttaatcgctg aattttatct ttaactagta aatcaaggtt catgactgct
25080

tcatcagcgc ttaggatttc ttcagaaaaa atttgtagcg ggttaatttt gtacttcgca
25140

ttagaagtta agtgcaagac tgcgccacct aaagcttcga caattttggt atattcattt
25200

tctggatcaa taatgtagag ctgatagtct tgaatggcat agcgtaaaat tttttggatc
25260

atataggtag tcttgccgac ccctgaggtc ccgataatca cttgattttg atttagggtc
25320

ttgttgcgat ccaacatatc tacggcaatt aagctgttgg tatctttgtt aaccccttca
25380

atatcagatc taggcttcaa gtccaaaatc tccgcgtcgt caaagggaaa catactgctg
25440

gcgacttcgg tattactttc tttataggtg taatcaccta atagattctc gttaattggc
25500

atggttgacc agaaagcttg aaacgttgct ttaacgggta ccagcggctt catctgtagc
25560

ttaatcaacg tattcttaac gttttcggtt agatcgtcga gttctgctaa actgttagtt
25620

cgcagataaa tgagcatatg ttggtacaca aatgtggtag aattatcaag gtatttatct
25680

agttgcatgt tggctgaatt aatataattt tgcagaatct tctttttata cgggtcgaac
25740

gtattgagct tctgcgcttc cttattttga attgtcttct tgtaataggt aatcattgaa
25800

ttgttactgg cgctgtcgat atattgcaca atatcgataa cgcctttttt gcgctttaac
25860

tctgatagcc agttgccata cactcgcttg ggatagtcag cgatcgctaa cacccgaata
25920

aaattctcgc ccgactggac gtaggtgggg tactgttccc agctgaacgg aaatagtgaa
25980

tgcaagctgt cgcgatcaat caacttggta aaatcctcaa tctccggttt gggctcgcta
26040

actgctagtt catgattact tggctgtctt tcttttttag ttggcataaa caaatcaatg
26100

atcttatcac caaaacccat actatttccc cctatccatt aaaattgatt aagttcttta
26160

aaatttcttg gacttcctga ctagtcaaaa ttttggccgt cacatcaaag tctgttaacg
26220

cattttcaat atcccgttta acctgttcga tcttttcgtc taaatgagcg accgctaagt
26280

ttaagctttc agcgcttttg tccttaattg gcactttaac gattagcaga tgttgtttag
26340

tcgtcatgtt tttggattct tggactttag taaaatgatc caagtaactg gctatgagtt
26400

ggattttgaa ctgttgctcc gggtggtctt tttgtaaggc gagataccgc cgtttgagac
26460

cattgagata agtcgtcata ttgacaggaa tcgtcggttc taagaactgt aacgtgtcat
26520

caacgccttc acctaaggta ctcatgagaa acgcgccata gtcctcaaag acgtcctgtt
26580

gttcggtttc attaagtaga tcaaggttga tcccgctaac ttccaaaatg ccaacgagat
26640

tacccgtttt ggtaacttcg taattaccat acatacccac taccaagctc atgtcatcaa
26700

tggtttcttt gccaccattg attttaggcg gttggtaatc ccaatcgagc ttcactgatt
26760

tattggcggt cttgctcttc ttcaaacgca tttagttcac ctcgcttctt cggttttaag
26820

taaaacaatt tctgactatt ggcataacgg cgtttcaact taaagttatc ccgcacctta
26880

atgttcttcc gcgacgcaac tggcctaatt gttaaaatag ccatgacaat cgtcatggct
26940

aagacaacaa tcactatttt tatcaggact aaaatcagac catagggtgg aatcgcaata
27000

aaaattaagc ccactagtcc tgccagtgcc gcatagccaa aatccttcaa ggtgtagtct
27060

ttccaaatcc cgtaatcttt atctacgttc tctgggaaga taaattcttt ccctttcttc
27120

atttggcaag ctccttttta aattaagtga ataggctgta gacccaaggc agtagccaga
27180

taaccgctgc cgctagggcc actaaaccag ccacccgacc aactgcgtga tatacctctg
27240

atttctctcg cggatcgtct gcgtcaggca ttcttttgat aataataaat agcgcgacac
27300

aaatcccaac taaaacgacc aagccagtta aaataccttg aatcgtctta ccagcgctag
27360

ttagtttgct ttgaacttca ccaccgtccg ctgccaaaac aacctgagta ttcatcaggc
27420

ccaagtagat cgcagtagct ccagtagttg ctaacgcttt tactttgctg aacttcatgt
27480

tattcctcct tcctatcgtc ttctgatgtt cgtaattgaa tttgttctgc ctgcgtttct
27540

tctggctcac gctgatagaa cttgttcaat tgttcctcgg caatttgctg gctcggtaat
27600

gggtcggtgg ctaatttgac gcggccgttt tgactcgtag ctaagtaata ctctttttct
27660

gactggccta attcttgggt ctctaagaca tgcaaattat gttcgtaacg gcctaaggcg
27720

ttatcaactt ctaagccata gaagcctaag aaaccttcta agaagctctc accaaaaaag
27780

gtcatttcgt ttttgatctg cttatattga tgggtcggtt tatcactcac caatttttca
27840

gcctttttag cattttgact gtcttgatgg cgattaaacc aaccactaag ctttaaggga
27900

tcagttttca actgacgttt aacggtcttc ttaatcattg ctcgacgaac ttcatcgatt
27960

ggctggccgt cttgatctaa aattgcttgt aactgttttg gatcagtcaa tgcttggtca
28020

tcatcaacca caatcgcctt ttgatcatca tgaactagct tgccattaat gccttgccag
28080

ctttctactt tcattggatc acctccttat gttgtttgtt gggttagaaa tattaaacaa
28140

ctagctttcc cctaacccgc ccggtaaaat ggattattag gagggtgaca cctagaaggc
28200

gtatttctag gtgctgatcc ccgttagcaa cttcactgct gacgaggacg ccctcttttt
28260

ttatgtggtt tttaacccac tctatccctt catcagatat taatttattg gtacctgaac
28320

caagcaaaaa ttgatcaggg aactctggat gtcgtttgat ccagttgctg acataatttc
28380

ggccacgccc cagcttatga ttagcttgcg ttaggctgta ccattcctga ttgcctgtca
28440

catgtaagtt ctcctttctt aatttagtca tagtttagca ctaaaaggtg taacttacac
28500

catttatttt ttttacattt aggttacaaa tgcatgcccc catcttcatg ccggtttcga
28560

gtttggtgtt gccgttgctt tttcgtcatt tcccactcag tctcctttag tccacgtagt
28620

tgctgctttc ttagatagtc cgtgttcttc gcatataaaa tggttattaa ggcttcgtcc
28680

aaaatattag tcaggtagtt tttcgggcta gttgggtaat aattaatggc ctgatagtcc
28740

ttaatctggt cttgcttata ttgatcaaac cgtgttgtct gttgtttcaa acgggcttgc
28800

accatttcaa tttgtttgtg atccaactta ggatcttggc cacattcgcc gataaacagt
28860

tgccgggtgc catcatgttt taagacccgt tgcagtcgat gatcctgaat atcttttaat
28920

tccattttgc ctaacaggta atttaaaccg gcttggtgtt gctgactagt aaagacgacc
28980

ggtggctgct gttgtaattg actgagatca ctggcgctta atggctttaa ttctggatca
29040

tagtaaacca cagcggtata gatttgctcc ttgtcaatcg gctctagctt atcgaggtca
29100

ctatccggaa aagcagtcgt tagaatgctg tcgtattggg tttgcaccac ggatttaacc
29160

tcccgaatga cccgtaaatc tttcaccgct ttcgaaatga attcgagttc gttaggttca
29220

taagcggcta gtatgccccg atcagtatgc ttcaaatcgt ctaactggtg gccatgtttg
29280

gctaacaccg cggttaattg cttctcaatt gtttgggctt gctgattaac taatttccag
29340

ctcacatatg ggcgtttggt aaacgtcaat aattgttggg ttaataagtc atttcgaata
29400

tccgctaacc gttcagtcaa ttcactatct ttaaaaatcg tttgttcgct attagtttct
29460

ttaattagtt cccgccgttc agcggccgtc gtccgttcaa aatcaagttc cggatataat
29520

tttttagttg ctcggtccac cactttattt aacaactgat tagcttgtgc tagtgatgtg
29580

gtttgctggt caatcgtgag taatttcttg gtcacatctt cgccaatcgc atgtttaatt
29640

aataagctgt ttttccaatt aaacagcatg cattgcttgt catctaagtg ttctaaatcg
29700

acataagttt tcagttgttg acttaggtgg ctaatcgtgt gcttttcgga aaatgataac
29760

tcgtcagtta atgcgtcaaa atgttcatga tttcggatct tttcgttaat attatgatac
29820

ttagcttgag tgcttctttg tgctctaact tgttgattaa acgcttttct attttgtctc
29880

tgactattaa tcccttcatg ttgggtaggt gtctcttgga ttccttgatc gacaaatgat
29940

ttttcgctaa ttcgatcggg aatatttttt tgtgctaaag actgattaac acttgttgcc
30000

caattatgcc gccactcatt aattttttcc tttttatccc aatcaaccaa ccaaattttt
30060

ctttgttttg gaaacccgct tttggttaaa agttgcttgc cattttcatc tttaatgtac
30120

tgcgtctttg cttttaatcc ccaagtacca tcggggttaa agggccgatt agttaacatc
30180

acatgagcat gcggattatc cgggtgctca cgatgaattg cgacatcagc taccatacct
30240

tgatcgacaa aattctcttg cacgtattta gttaataatt ctttttgttc atctgtactt
30300

aactcaattg gtaaagctac gttaaactct ttggcatatc gtgagtttga tttacgatct
30360

ttcttttcaa cttcattcca caacttctcc cgatcactcg cccattttgg tgcattcttt
30420

ggtgtcaaaa taaagctttc tggcataacc gaacgcgcat aaaaatagct tcggccttct
30480

ttttgatcga ataacttttc accacttcga taagcagctc cagcaatggc actacgtcct
30540

ttaccagcac taatattttg aaaactcatg tgaaaaattg ccatgttggt caccgtcttc
30600

ctttgattat atgagtttga ctttgttgtc gccaacggcg acttctaata caggttttaa
30660

tggggttaac acaacataga atttattcta tgtgtaagtg cgcattgacc tcgtttcact
30720

cggtctgctg attatcctga atttactttt agtgtatgcc ttccgcttcg ctatttcaaa
30780

ttttatactt tgacttaacg tttcttatat gatatagttt cggtgattat ttctaatatg
30840

attggaggga tcgttatgtc tcaaagtaac ttagaaaaac aagaagctaa attaaaagcc
30900

cttaatcaaa aaattaagga cgaaaaaaat aagattgaac aacggctagg taaacaaatc
30960

atcagtcaag ccaatttaga ttatgctaat ttgtctaacg atcagattaa gcttttagcc
31020

aagcaatttt ctgaattttt aaaggtaaag tccgtagatc gttagccata caagatgggg
31080

aaattccatg tctaataaat atgaaaaact agtcgagcag caagcgcgtt taaaacaaaa
31140

aattgagcgg gaagatttta aattacgaca atctaaatac tatgaaaatc ggcaagcccg
31200

caaagcccgt tctcgccgat taattcaaaa aggggcttta ttagaaaagt actttcaagc
31260

taataacctc tcggtcgaac aaaccgaaga acttttaaaa acatttgctg actatgttaa
31320

cgcccataaa ccggataaat taaaaaacga tcaacctaat aactaggccg atcgttttta
31380

ttttcaggat tgtttttggg cttaatttct ggattgtgtt tagcaaagtc ttttaactgt
31440

ttttgaattc tttgtgtaag ctcggcttta ctttctttgt gcttcctttt acttgatcgc
31500

tgttgaatta tcttttgacc cttacttctt ctttttgatt ttagatcaag cgtaaaatct
31560

gaaattttat tttgatctag cttacttaaa tgaccgattt ctttaaagtt taaaccggct
31620

ttgggaaagc gataaatatt accaagatcg acccaggaag gtttcttcaa cccagcggat
31680

tgccaatctt gaatcggata atattgttgc ttgatataag ccgacttctt ttcatactga
31740

agtaatttta aaagcctcga ccgtctgctc tgatacccga cgaattaaaa ctggtcgaga
31800

cttgccaccg ttagtttggg gtcaatggaa catccaaacg aaaagtagcg gtagaaaggc
31860

gaataaccag gacttaaatt agtgaggttg ttcgtttttt cgtttgagct tttttagttt
31920

caaccgattc acttcgcggt aaaaagttcc tgaggaaatt tggttagtgg ctaaaatttc
31980

tttaactgaa tagtcaccac tttgatacat ccgaatggca atatccaact tttcttgggc
32040

gattcgtgga cggccaactg ttacgccacg tttgcggcta gccgcaatcc cctcctttac
32100

tcgatcagcc agtagattcc gctccagttc tgcaatcaca ctcatcattt gcagcatggc
32160

ccgaccggta ggtgtttcgg tgtccatctg ctcctttaaa gaaacaaact taatttgttt
32220

ttggtgcagt aattgaatca aatttaaaat atccaacgta ttgcggccca accgggaaat
32280

gctttcaacg accacaacat cgtgagcccg aatggtctgg agaagctggg taattccggg
32340

tcttttttgt cgggttccag taaacttttc ttgaatgatt ttttcggcac catagggcgt
32400

taattgatcg atttggcgat taaggttctg atcgtctttt gaaacgcggg catatccaat
32460

aatcatgagt gactccttag ttttttgata gccaagggaa aattagctat cttttggcaa
32520

tggtttatta taaacttttt gataatgagt ttttgaaaac tcaaaaaccg gttaaatcaa
32580

cctaatcaaa gtatagcgaa acctttccaa aaactatagt tttttaaaaa cattggagaa
32640

taatatgccg actaatccgc atcaccaaca aagcttcgga acttttgagg gcgtcccttc
32700

tgccgaccaa ctgcggctgt attttcaatt aactgatttt gatcgcgcgt taattgatga
32760

aatgcgttcc gcaacgacta agttaggctt tgccgttcaa ttgagctcag ttagatttct
32820

aggaactttt ccaactaatc tacaacaagt cccagctgag gtaattgatt atttagcgaa
32880

acagttaact attgatggtc gagcattagc aggctacacg cgaaaaatga ccattagcca
32940

acacatgacg ctaattaagc gaaaatatca ttatcaagcc tttactgatc cagcagtcaa
33000

agcgagcctg aacgactggc tgcttgagcg agcgcgtttt acaaccgaaa ccgaaaaact
33060

actttttgat atgctgctta aaaaatgttt ggatgaaaaa attctcttac caggggtgac
33120

cacctttgag cgctttatta atggggtcac ggaagttgcc gccgaccttt tggatagtca
33180

attagtggcc gttccttcag tggaagaaac ggaccgactt ttaaatctgt taactgccgt
33240

tggagaaccc atctatgggg ccaccattaa aatggatctc ttaagaaatc ctttgatgga
33300

cgaaagccgc aaagaaatta accgtggctt tactcgatta aaacagtttc aacaatttaa
33360

aactgaggat tggcaattag gcgcaattcc agcaggaaag ttaaagaatc tggccaacta
33420

tgcttttaaa gccaaggcca gtctgattaa acggatgtcc gtcaaaagac aaacggcgtt
33480

attggtcgct tttgtttccg agtatcgcaa acgagcgatg gatgaacaat tactagctct
33540

ttcgcgcttc tacgagacgc tttttaagcg ggccaaaaaa aaagaagcca aggaacggct
33600

gcgaaccatt aaagatcttg atcatgcagc tttaacgctc tcccaaattg tccggttatt
33660

gatggacgaa acgatcagca gtcaagatct cagaaacaaa gttttagcaa cttatccacc
33720

tgaaataatt gagtcagccg ttactcaggt taatcagtta gtgcgtaatg agcgagaacc
33780

aattgccatt gaagaactgt taaatgctta tcgaaaattc cggaaattca ttccggatat
33840

tttagccacc attgtttttg aaggaaccat caacgggcaa gattgcctga tagtttggga
33900

gtttattcgc caacgatttc cgcgcccggt tacctatcgg atgtttgaac agatcgccga
33960

aaaactaccg aaaaaatggc gttattatat tcgtgagaac cccaaagtaa ccaatcagtg
34020

tgtgttgatt gccggcatcg agcttctggt tcatggtctt aagcgccatg atatttacgt
34080

ccggcaaagt aaccgttatc ttgatcccat ggcctgctta attgataagt ctacttggcg
34140

tcaacaaaag gctgttttga ttcaacaact tgatttacca cagactggtc aagcagccgt
34200

tgaagcggtg aaacaggatt tggctttatc ctatgccgag accctaaaaa attgggctga
34260

ttcagagatg gcccaggtca aagaggttga tggccaagaa aaaattattg tggccaaatt
34320

acgcaaagcc cgtgaacaaa aggacgaagc ggcctttaaa agtcgggttc ggcatctaat
34380

tcccaaaatt gatttatcgg atattctctt ggaggttaac caacagttac agctaacgca
34440

atgttttagt cacgttagtg aaagtgagac gaagatgaaa cacgttgata ttagtatttt
34500

agcggtttta ttggctgagg cttgtaacat tggcctttcg ccagtggcta aaaacagttc
34560

aaatagtcta aaatacgatc ggttaatgta cgtggaccat caatatttgc gaattgatac
34620

ccttgccgcc gccaatcgcc gcattattaa cgcgcataaa aaattaaaaa gtgcgctcat
34680

ctggggcgac ggtcaaatgg cttcggccga cggaattcgt tacgttaccc cccaaaggtc
34740

tttatattct cgcagcaatc caaaatactt tggccgcggt cgcgggatta ctttttacaa
34800

ttttgtttct gatcaataca tcggctttca tggcatggtg gttgcaggga ccttacgaga
34860

ttctctttac ctattagaag gctttttgaa tcaaggcagc agtttggaac ccactcagat
34920

tatgaccgac accgctggtt atagtgattt agtttttggt ttatttggcc tattaggttt
34980

tcagttcagt ccccgaattg ctaatagtca gggaaccaaa ctatggcgga ttgattcgac
35040

cgctgattat caagtcttaa atggggtgtc tcaaaaccgc attaatttaa agttgattga
35100

agaacattgg gaagatattt tacgcgttgc cggttcgtta aagtctggta aggtcaatgc
35160

gacagaatta actcaggcct tgcagcgcga agggcaccca accgctttgg gtaaagcgat
35220

tactgaatat ggcaaagtct ataaaacgaa acatcaatta cgctatcttt cagatgagct
35280

ttatgcacgg caaattttgg aacagctaaa taaaggtgaa gcccggcacg cgctttgtcg
35340

aaacatcttt tatggtcgga aagggaagct ttatcaaacg tattttgatg gtatggaaga
35400

acaattaaac gctctcagtt tagtcacgaa tgccattatt tattggaaca cggtttacct
35460

ggaaaagatc gtgacgcaga tgcaatcaga agggtttgat tgttctgacg acatgatcgg
35520

caaattatcg ccactgatgt ttgaacacat taactttgtc ggtaagtaca cgttccaata
35580

taatgaagcg ttagaaaatg gtcatttaag accattggat gaagcagacg aaaaataaga
35640

ttgttcggtt tttaaagtct accgctactt ttcgtacgaa tgttccagag ctctttttaa
35700

agaaagccaa tataaaaaat cagaaacgct gttaaatcaa cgtttctgat tttttgttcg
35760

ggttcctacc gctacttttc gtatggatgt tccattgacc ccgtttctac aaaactaacg
35820

tataggctga ctaagtcatt agtcctcatc tggatcgttg agccaatcag cgacctcttt
35880

agcgtctttg aactcggtga ctggtgtccc ttcggtcgcc tttaaaaagc gtaaattagc
35940

tctttcttct tcggttaaag acgcattaaa aggtaaagca ccattagcaa caatccgctt
36000

gtaaaacatg ttaatggccg tggttggatt taagcccaat tcgcttaaaa ctgcttcggt
36060

atcatcggc
36069

<210> 4

<211> 3015

<212> DNA

<213> Artificial

<220>

<221>  misc_feature

<223>  plasmid p706 with pWCFS101 replicon



<400>  4
aattcctagt ttggcgaccc ggaacacgtg agttaatctt gaatattcgt atttactaga
60

catagtttaa agcttgagtt agtaagcgtc aagcccttag ttttagtaaa tacataaaag
120

attagctctt ctcacgtggt tggatgagag gagcttttta gtttggctga tagaaaagtt
180

ttagttgatc gatcgcagtc gggaaaagta cgaccatggc gagaacataa gttagagaat
240

ttacagtatg gtgattattt acaaatgttg cactataaga aatcccatcg agttaaagag
300

tgtggtgaag tattacgttt tgtggaagat aaaaatggtc acaaaaaatt ggctcagact
360

tggttttgcc attcccgttt gtgtccgtta tgtaattggc ggcggtcaat gaaacaatct
420

aaccagttaa ctcaaatttt gacagaagca gttaaacagc gaaaaacggg tcggttcttg
480

tttttaacgt tgacggtgaa gaatactaca ggggatttgt tgaagagtga gttacggcag
540

atgggacgag ccattgcaaa gatctttcag tataaaaaag tggctaaaaa tttgttgggt
600

tatgtacgtt caactgaggt taccattaat cacgaagcag atcagccgat gtatcaccac
660

catatgcatg ttttgctttt tatgaaatcg agttatttta caggaactga taattatatt
720

tcacaagcgg aatggaccgg gtattggcaa cgagcgatga aattagatta tgtgccggtt
780

gtgaatgttg aagcggttaa accgaatgtg aaacgccaga aaaattcctt gctggctagt
840

gcccaagaaa cggctaaata tcaggtaaag tccaaagata ttttaactaa taatcaagaa
900

caagatctac aagtaattga tgatttggaa caagctttgg ctggttcccg ccaaattagt
960

tatggtgggt tactgaaaga aattcgcaaa cagttgcaat tggaagacgt tgaaaatggt
1020

gatttgatta atacggatag tgatgatcaa aaggttgatc aagtggtgcg tgagattgtt
1080

gctaaatggg attatcagcg taagaattat tttgtatggt aattttagaa aaaggacgga
1140

tgactgatcc gtcctttttg tttttgatgc agtgttttct gtttctagtc gacggcaata
1200

gttaccctta ttatcaagat aagaaagaaa aggatttttc gctacgctca aatcctttaa
1260

aaaaacacaa aagaccacat tttttaatgt ggtctttatt cttcaactaa agcacccatt
1320

agttcaacaa acgaaaattg gataaagtgg gatattttta aaatatatat ttatgttaca
1380

gtaatattga cttttaaaaa aggattgatt ctaatgaaga aagcagacaa gtaagcctcc
1440

taaattcact ttagataaaa atttaggagg catatcaaat gaactttaat aaaattgatt
1500

tagacaattg gaagagaaaa gagatattta atcattattt gaaccaacaa acgactttta
1560

gtataaccac agaaattgat attagtgttt tataccgaaa cataaaacaa gaaggatata
1620

aattttaccc tgcatttatt ttcttagtga caagggtgat aaactcaaat acagctttta
1680

gaactggtta caatagcgac ggagagttag gttattggga taagttagag ccactttata
1740

caatttttga tggtgtatct aaaacattct ctggtatttg gactcctgta aagaatgact
1800

tcaaagagtt ttatgattta tacctttctg atgtagagaa atataatggt tcggggaaat
1860

tgtttcccaa aacacctata cctgaaaatg ctttttctct ttctattatt ccatggactt
1920

catttactgg gtttaactta aatatcaata ataatagtaa ttaccttcta cccattatta
1980

cagcaggaaa attcattaat aaaggtaatt caatatattt accgctatct ttacaggtac
2040

atcattctgt ttgtgatggt tatcatgcag gattgtttat gaactctatt caggaattgt
2100

cagataggcc taatgactgg cttttataat atgagataat gccgactgta ctttttacag
2160

tcggttttct aatgtcacta acctgccccg ttagttgaag aaggttttta tattacagct
2220

ccaagatctc agctgggatc cagtactctg caggaattct ctagagagct caagcttctc
2280

gactagaatg acttcaattt atttggagtg taagtgcgca ttatcatgta gtgcgcatta
2340

tcatgtagtg cgcattatca tgtagtgcgc attatcatgt agtgcgtatt atcatgtagt
2400

gcgcacttac acacaacatg aagttgtgtt gtgctaaacc catcaaaacc tgcatcagat
2460

ttcgcgttgc tcaaacgcaa ctgacttgcg tcagtttgga acattcaaaa ataaataagt
2520

tcagtcgcta gctccttcga acttttttat ttttgaacgt taattttaaa ggctcttatt
2580

tgcgttctaa gcgattttag ctaacagtga tccatctaac tgtctgtcaa cggtaaatcg
2640

aattaaaggg acttactgct ttagcagtta gtcccttttt gaggctttaa ggagttgact
2700

gactaaccaa acccagacac tgaattggct gaaagccaaa gttccctaaa ctttgctttc
2760

ctgcctcacg gcgagtgcgg ggtgagtttg agcgggagct cccgctcaat tatgcggtca
2820

agctggcaca gcttggactg ggttcggggc gtcagcgccc gagggcttag aaactggtcg
2880

attttgacca gtttcttctt atcttgatac tattagaaac aacgtcattt taaaaaaccg
2940

ggataaaccc ttgacacaac tgggcttagg cgtattatga gtttataaaa tgaataaaga
3000

aaaaacccac gtgag
3015

<210> 5

<211> 3463

<212> DNA

<213> Artificial

<220>

<221> misc_feature

<223> plasmid p707 with replicon from pWCFS102

<400> 5
attcaagaaa ggaatcagag caaaaaaata agtgagaact cattcccttc aagaaatacg
60

agttctcact aatgtttgtt ctgtcaaggc gattataaca aatggcaaaa gaaaaggcga
120

gatacttcac ttttttactt tatccagaat cgataccaca agattggcaa acaaaacttg
180

aaactttagg cgttccaatt gcaattagcc cgcttcatga taaagatttg agcagtgttg
240

aggggcaaaa gtataaaaaa gcccattatc acgttattta tatctctaaa aatcctgtca
300

cagcagaaag cgttcgcttg aaaatcaagc gtgctttagg cgataagagt gttgctaaag
360

tacaaattgt tgtccagagc atggaaaata tgtatttgta tctgacacat gaatctaaag
420

acgctattgc taaaaataag cacaagtaca gcaagcatga cattgctgta ctgaacaatt
480

ttgatattga tcgctatatt acgcttgatg ttgaagataa agacgacatg ctgaatgatg
540

tttgcgattt gattgatgac cataatttgg cgaatatgcg tgaactgaga cgctttttaa
600

aagctcatgg ttcagaatat ggcatgcccg gtattaaagt cgttaattcg gttttacgtg
660

ctcatactgg actgataagg ctgtatttcg atgctgttta tcaggaacgc aagtatggca
720

gaggcgatat aaacaaagag actggcgaga tacaagatta attagcgaat ggaaagtagg
780

tgttcgattt gagcaccttt tttgttgtcg gctagccgac ttctgataca agtctttagc
840

acaataacac aatcaacttt agttgttgtg tgtaagtgcg cccttaggaa tataatttga
900

atatatttca gattttcaat ctgactgcta ctatcatcaa gataagaaat aactaatttg
960

tgtcaaaaat tttctctaaa tctgtttcat caacattttc ttcaatttcc aaatctctat
1020

acatctcaac aaaaatattc actaattttt cattagcaag cccgtcatca accgatgaat
1080

ttgacttata gctataaaaa tttttataac ttttaataac atctggcgaa tcaaaaaaaa
1140

cagcaggaac actattaaac gctctcttaa attcagtgct agaaaaatca tatcttgaac
1200

caatcaattg aactaaaact tcatatttct tcatccaaat tcttcgttca tgttcctgaa
1260

ttgctaaggt attttggaaa ttttcattat ccgattctaa ttgtaattga aattttttac
1320

tccttttgtc attaaaaaat gagtaaaaca aagtaatagt cacacctacc aaacctaaaa
1380

taccacctac caaattaatt cctcctaaat ttttatttat attttatttt acgttatttt
1440

aagacgattt tagtattttt taataaaaat tatttataaa tcaaaaagta ctcttaaaac
1500

acagatataa gcaattaaga aaataaaaat ctcaaaaaac aaaatctcta ttttttaaaa
1560

atcgctatag ttcttgtggt gactgaaaaa acgagaatta atttgaaatt ggtgtttttt
1620

gatatgaaaa taaagtttag tcctcttttt gttttgaata gttctagtcg acggcaatag
1680

ttacccttat tatcaagata agaaagaaaa ggatttttcg ctacgctcaa atcctttaaa
1740

aaaacacaaa agaccacatt ttttaatgtg gtctttattc ttcaactaaa gcacccatta
1800

gttcaacaaa cgaaaattgg ataaagtggg atatttttaa aatatatatt tatgttacag
1860

taatattgac ttttaaaaaa ggattgattc taatgaagaa agcagacaag taagcctcct
1920

aaattcactt tagataaaaa tttaggaggc atatcaaatg aactttaata aaattgattt
1980

agacaattgg aagagaaaag agatatttaa tcattatttg aaccaacaaa cgacttttag
2040

tataaccaca gaaattgata ttagtgtttt ataccgaaac ataaaacaag aaggatataa
2100

attttaccct gcatttattt tcttagtgac aagggtgata aactcaaata cagcttttag
2160

aactggttac aatagcgacg gagagttagg ttattgggat aagttagagc cactttatac
2220

aatttttgat ggtgtatcta aaacattctc tggtatttgg actcctgtaa agaatgactt
2280

caaagagttt tatgatttat acctttctga tgtagagaaa tataatggtt cggggaaatt
2340

gtttcccaaa acacctatac ctgaaaatgc tttttctctt tctattattc catggacttc
2400

atttactggg tttaacttaa atatcaataa taatagtaat taccttctac ccattattac
2460

agcaggaaaa ttcattaata aaggtaattc aatatattta ccgctatctt tacaggtaca
2520

tcattctgtt tgtgatggtt atcatgcagg attgtttatg aactctattc aggaattgtc
2580

agataggcct aatgactggc ttttataata tgagataatg ccgactgtac tttttacagt
2640

cggttttcta atgtcactaa cctgccccgt tagttgaaga aggtttttat attacagctc
2700

caagatctca gctgggatcc agtactctgc aggaattctc tagagagctc aagcttctcg
2760

actagaacat catattttgc gttctaagcg attttagcta acagtgagct atttaactgt
2820

ctgtcaacgg taaatcgact tagaggggct tggagagcct tacaggcgat attagcccct
2880

cttggaggct ttaaggagtt gatagactgt acgaaaccta aagcctgatg agcaggaaac
2940

aagcccttgt ttccccgagc ccagtggcgg cgaacgatag cggtcaagct ggctcagctt
3000

ggtctgggtt tggggcgaca gcgcccaagg gttttaaaaa gacgtttcag cgatttttgc
3060

tgatccgtct tttttggttt gtgttgtaaa taaattttta gcgctgaggg gggcaaggaa
3120

gcaaattttg cgaccgtact acgacccccc ttttaagtgc cgagtgccaa aattgaattt
3180

ttcgggacct tatccctact ctcccaagga ctacagcgat taaaaaaaaa tcaacaaatc
3240

gccaacaaaa ttggcaaaaa aacgtgttga tgtaaaataa cttgacaaaa aaagcgaata
3300

aaagtaaaat ttgtttacaa tatttaagag gagaatatta tggctgaaaa taaaaaaaga
3360

gtttcgattg ggttgacgga taaacagtat gaattagttc aaaaattggc taaagaaaaa
3420

ggcttttcga agtctgcaat atttgtttta gctttagaag aat
3463

<210> 6

<211> 4246

<212> DNA

<213> Artificial

<220>

<221>  misc_feature

<223>  plasmid p701 with replicon from pWCF103



<400>  6
tcgagaagct tgagctctct agagaattcc tgcagagtac tggatcccag ctgagatctt
60

ggagctgtaa tataaaaacc ttcttcaact aacggggcag gttagtgaca ttagaaaacc
120

gactgtaaaa agtacagtcg gcattatctc atattataaa agccagtcat taggcctatc
180

tgacaattcc tgaatagagt tcataaacaa tcctgcatga taaccatcac aaacagaatg
240

atgtacctgt aaagatagcg gtaaatatat tgaattacct ttattaatga attttcctgc
300

tgtaataatg ggtagaaggt aattactatt attattgata tttaagttaa acccagtaaa
360

tgaagtccat ggaataatag aaagagaaaa agcattttca ggtataggtg ttttgggaaa
420

caatttcccc gaaccattat atttctctac atcagaaagg tataaatcat aaaactcttt
480

gaagtcattc tttacaggag tccaaatacc agagaatgtt ttagatacac catcaaaaat
540

tgtataaagt ggctctaact tatcccaata acctaactct ccgtcgctat tgtaaccagt
600

tctaaaagct gtatttgagt ttatcaccct tgtcactaag aaaataaatg cagggtaaaa
660

tttatatcct tcttgtttta tgtttcggta taaaacacta atatcaattt ctgtggttat
720

actaaaagtc gtttgttggt tcaaataatg attaaatatc tcttttctct tccaattgtc
780

taaatcaatt ttattaaagt tcatttgata tgcctcctaa atttttatct aaagtgaatt
840

taggaggctt acttgtctgc tttcttcatt agaatcaatc cttttttaaa agtcaatatt
900

actgtaacat aaatatatat tttaaaaata tcccacttta tccaattttc gtttgttgaa
960

ctaatgggtg ctttagttga agaataaaga ccacattaaa aaatgtggtc ttttgtgttt
1020

ttttaaagga tttgagcgta gcgaaaaatc cttttctttc ttatcttgat aataagggta
1080

actattgccg tcgagtaagc aaagcctgta tgtaatgatt tcagaaaagc cctctattcc
1140

gcataataga ggactttttt gtgtgcattc tgcttaattt aaacggacaa gtatacatct
1200

aaaaggtgaa cgtgtcgtct gagagcacgt gattatttac tttttacttt gttagcatcg
1260

ccggtttcca aagtctgtat ttgagtcgta agcgtatttt gttcagactc agttaacgat
1320

tcaaaaaaag ttttttgtaa ttgggctaat ccgttttgtt gagttgagct ataccctaaa
1380

attaccatag ctttcataaa gttttttaga tgattgtcaa ttctgagcgt agtatcaaaa
1440

gtaacaccat ctatttgttt agacttcgtt ttgtttgatt cgtttagaga agatatggaa
1500

atttcttttt ggggctgggg cgccgatttt ctaggaatta ttttgctttt atttgagtta
1560

ggattgtgta aaagttccac catgctagtc cgccctttca ataatttctt gtgccacaga
1620

atcgtataat ctaattattt ttttatcgaa ctgatctttt agaaatacac ctctgattga
1680

atatcttttg attctttcca tattacgaat tagggttttt aacatgtttt ctttcccaaa
1740

ttcttcttct gctacttcta atgtgctgtg atcaacaggc gccccattct ttaatagcac
1800

aggaagtata ccgagtaaat tcaaactagg tgcttcgtat tcatcgatta cctgttcctg
1860

tatatatttt agaaacgatt cggcaccctg aagactgtgc tcttgagttt gaaggataat
1920

cagaacccaa tctgaagcgt ataaggcgct atcagaaatt aaggaaatag tcggtggcaa
1980

atcaaatacc acaaaatcat atttttcttt tagtgaagca agtagcttgc taaaatatgt
2040

aactctatct ttataattat ttttaaactt tttttccata atccgtgggt acaatgaaaa
2100

atctgcgctt gatggaataa agtcaatgtt tttatccaaa gtaaccaacg atcttgataa
2160

gtcctcttct tggattgatg caagcaaggt ttgattaaat tcaatatcat cgtggcctaa
2220

attagccgcg gttttaaagt aaatatctgt agcgtttgct tgaggatcga aatcaattaa
2280

tagcgttttt ttaccagcac gagctagtgc caggcatgca agcgtggcat ttgttgtttt
2340

accggtgcca cctttaaaat tcccaaaagt aattacttca cccattatac tgacctccaa
2400

ttaagtatcc taatatacaa ggatacttgg atacaaggat gataacatac aaagatacaa
2460

ggatacaagg atacaaggat acatttcttc tcaaaagccg ttaaattaag attgactgca
2520

agtatcctaa tatacttaga tacttagata caaggatact tagatacaag gatatgaata
2580

tacaaggata caaggataca aggatacata aaaagaaatg ttgatttatc accaaacaac
2640

gagtaatatt ggttacataa aatagaacat aaaaaaacac ccaccgacgg gaatcggtga
2700

gcgccacata aaagtcaact catttactga ctattatatc atggccggtg cgatttttaa
2760

agccctaggg gacaagctgg ggtctaaatc caaggggaca cgtttgccag tgcgacttca
2820

ataagtctgg ctataccaca acaaggtcat ctgtacggct gggtggtgaa tggtgagcgc
2880

gaccattaac ggcgcgggtg gtaaaacgaa gcttcggctt ggtgccactg attagttggt
2940

gatcgaacaa acataaatac gtattgccaa cgcctactag ggcgtttttt agtaggttaa
3000

accgaaaacg taggtttata atgagtggtg gtagcaatac cgcaattgta cagacaagcg
3060

acgggcgtta acgaccgacg aactaagggg aaacttttag tagtagaatt gtactctggt
3120

tcagttattc caaatagctg attccaggga acaattctgc gctcaaaacg tcactccctc
3180

taaactgaat ggaaaaccgt aaaccgtcaa gtcaaattcc aaagaaaaaa agaaagttgg
3240

tggaattgtt ggaatgtaag gattacattg ttccattgaa tccagatgtg atcgttaaga
3300

agacaatcta tcggagttct gcaagaatta ctgagcgaca aaggaaccaa gtgaagagag
3360

ttttgcgtag tcaagggtat cagggaagaa ttagcagtat tcgatacact cgggtaaatt
3420

acaagtattc atttaaagta tcagtgaagt atcaggggaa tgtcggtaag tttttggtta
3480

atacaaagaa tgagacagtt gaatttaagg gaatggtcta ggagtaatac cagtaaattt
3540

cacaaaattt ctttccccaa ttaaagccaa tctgcgacct ataccacttc tttttggac
3600

aagttcacaa tcaaaaaata acggaatgtg gaagtaccgc ggtgaatgtg ggttacttgg
3660

gataagccgc cttatgtgaa gtagaaattg gtatactaaa aaaggctccc ggtttaatac
3720

tggaggcctt ttttctaggt tacatatttt gaggatgaat gttttttatc ttcgtagctt
3780

ttacttgggt gctgtaacat gactgcttag tttttggtgc cgtaccccgc aacaaatgcg
3840

gcgcttcatt ggcattatct taaatgctaa gtaccgagtt gagaaagatc acaaagatat
3900

tggcgtcata atcccacttg atgatgaaga acttaaaccg ctaatgacta aagccttgag
3960

acgctacttt aacgtcctaa gaagtaatga gaagcacatc aagaacgtgg aaaattacct
4020

gtacggcacc atgcaaaacc tatttggcgt ttggtggaat aaacaagcgg ctagagaata
4080

tgcggccaaa caccccgaag aagaaaagtc ggccgacaac gataacagtg ggttgtacta
4140

ctagtcctaa aaggctgcaa aatcctttat aagcggtttt aagacttact aacctcttta
4200

tgcttaagct agatttaaat ggcttaaaca gaagaatagg ggcttg
4246

<210> 7

<211> 32

<212> DNA

<213> Artificial

<220>

<221> misc_feature

<223> PCR primer

<400>  7
gccgcggtcg acaagcccct attcttctgt tt
32

<210>  8

<211>  32

<212>  DNA

<213>  Artificial

<220>

<221>  misc_feature

<223>  PCR primer

<400>  8
gccgcgctcg agtaagcaaa gcctgtatgt aa
32

<210>  9

<211>  29

<212>  DNA

<213>  Artificial

<220>

<221>  misc_feature

<223>  PCR primer

<400>  9
ccgcgcggta ccgttactgg gatcgtttg
29

<210>  10

<211>  29

<212>  DNA

<213>  Artificial

<220>

<221>  misc_feature

<223>  PCR primer



<400>  10
ccgcgcggat ccacattctt agggcatac
29


<210>  11

<211>  38

<212>  DNA

<213>  Artificial



<220>

<221>  misc_feature

<223>  PCR primer



<400>  11
ccgcgcggta ccattcttta tacatcaact gcaatcga
38


<210>  12

<211>  36

<212>  DNA

<213>  Artificial



<220>

<221>  misc_feature

<223>  PCR primer



<400>  12
ccgcgcggat ccaacaaaca gcgttacgat tagctg
36


<210>  13

<211>  35

<212>  DNA

<213>  Artificial

<220>

<221>  misc_feature

<223>  PCR primer

<400>  13
ccgcgcggta ccaaaatggg cgagtgatcg ggaga
35

<210>  14

<211>  37

<212>  DNA

<213>  Artificial

<220>

<221>  misc_feature

<223>  PCR primer

<400>  14
ccgcgcggat cccttgatcg acaaatgatt tttcgct
37

<210>  15

<211>  21

<212>  DNA

<213>  Artificial

<220>

<221>  misc_feature

<223>  PCR primer

<400>  15
ttcaccgaac actagggttg c
21

<210>  16

<211>  19

<212>  DNA

<213>  Artificial

<220>

<221>  misc_feature

<223>  PCR primer

<400>  16
attccgctgg cagcttaag
19

<210>  17

<211>  25

<212>  DNA

<213>  Artificial

<220>

<221>  misc_feature

<223>  PCR primer

<400>  17
tgcacactca agtctcgatt cagca
25

<210>  18

<211>  23

<212>  DNA

<213>  Artificial

<220>

<221>  misc_feature

<223>  PCR primer



<400>  18
tcaaatacag cttttagaac tgg
23


<210>  19

<211>  25

<212>  DNA

<213>  Artificial



<220>

<221>  misc_feature

<223>  PCR primer



<400>  19
accatcaaaa attgtataaa gtggc
25


<210>  20

<211>  24

<212>  DNA

<213>  Artificial



<220>

<221>  misc_feature

<223>  RT-PCR probe



<400>  20
gcgacggaga gttaggttat tggg
24